(19) Europäisches Patentamt
European Patent Office
Office européen des brevets

(11) Veröffentlichungsnummer: **0 067 382**
**B1**

# (12) EUROPÄISCHE PATENTSCHRIFT

(45) Veröffentlichungstag der Patentschrift: **04.12.85**

(21) Anmeldenummer: **82104909.5**

(22) Anmeldetag: **04.06.82**

(51) Int. Cl.[4]: **C 07 C 103/34**, C 07 D 307/14, C 07 D 307/52, C 07 D 231/12, C 07 D 249/08, A 01 N 37/18, A 01 N 37/46, A 01 N 43/00

(54) **Chloressigsäurecyclohexylamide, ihre Herstellung, ihre Verwendung zur Bekämpfung von Unkräutern und Mittel dafür.**

(30) Priorität: **15.06.81 DE 3123731**

(43) Veröffentlichungstag der Anmeldung: **22.12.82 Patentblatt 82/51**

(45) Bekanntmachung des Hinweises auf die Patenterteilung: **04.12.85 Patentblatt 85/49**

(84) Benannte Vertragsstaaten: **AT BE CH DE FR GB IT LI NL**

(56) Entgegenhaltungen:
**DE - A - 2 826 568**
**FR - A - 1 353 476**
**US - A - 3 007 786**
**US - A - 4 258 196**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Eicken, Karl, Dr., Waldstrasse 63, D-6706 Wachenheim (DE)**
Erfinder: **Goetz, Norbert, Dr., Schoefferstrasse 25, D-6520 Worms (DE)**
Erfinder: **Wuerzer, Bruno, Dr. Dipl.-Landwirt, Ruedigerstrasse 13, D-6701 Otterstadt (DE)**

EP 0 067 382 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft Chloressigsäurecycloalkylacetamide, Verfahren zu deren Herstellung, Herbizide, die die Verbindungen enthalten sowie Verfahren zur Bekämpfung unerwünschten Pflanzenwuchses mit diesen Verbindungen.

Chloressigsäurecycloalkylacetamide, wie z. B. das N-Allyl-chloressigsäurecyclohexylamid, sind bereits seit längerer Zeit als Herbizide bekannt (BE-A-532 981; US-A-3 007 786; FR-A-1 353 476). Außerdem ist aus der US-A-4 258 196 bekannt, daß N-Cyclohexenyl-chloressigsäureamide ebenfalls herbizid wirksam sind.

Es wurde nun gefunden, daß Chloressigsäurecyclohexylamide der Formel I

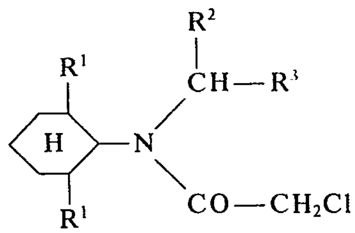

(I)

worin

$R^1$ Methyl oder Ethyl,
$R^2$ Wasserstoff oder $C_{1-3}$-Alkyl und
$R^3$ ein durch $C_{1-4}$-Alkoxi oder $C_{1-4}$-Alkoxicarbonyl substituiertes $C_{1-6}$-Alkyl, $C_{2-3}$-Alkenyl, $C_{2-3}$-Alkinyl, Furanyl, Tetrahydrofuranyl, gegebenenfalls durch Halogen substituiertes Phenyl, (Bis-$C_{1-6}$-alkoxi)-methyl, gegebenenfalls durch $C_{1-3}$-Alkyl substituiertes Dioxolanyl oder Dioxanyl oder – falls $R^2$ Wasserstoff bedeutet – auch $C_{1-8}$-Alkyloxi, $C_{3-6}$-Alkenyloxi, $C_{3-6}$-Alkinyloxi, $C_{3-6}$-Cycloalkylmethoxi, $C_{4-6}$-Cycloalkyloxi, $C_{1-6}$-Alkyloxiethoxi, Tetrahydrofuranylmethoxi, Tetrahydropyranylmethoxi oder Azol-1-yl

bedeuten, wesentlich verbesserte herbizide Eigenschaften besitzen und gleichzeitig noch einen hohen Verträglichkeitsgrad für Kulturpflanzen aufweisen.

Unter Alkyl oder Alkylrest einer Alkyloxi- oder Alkyloxicarbonylgruppe bei den Resten $R^2$ oder $R^3$ sind je nach Zahl der genannten Kohlenstoffatome beispielsweise folgende Gruppen zu verstehen: Methyl, Ethyl, n-Propyl, iso-Propyl, n-, iso-, sec.- oder tert.-Butyl, Pentyl oder Hexyl und ihre Isomeren.

Unter Alkenyl ist je nach Zahl der genannten Kohlenstoffatome bei $R^3$ insbesondere Vinyl, 1-Methylvinyl-1 oder 2-Methylvinyl-1 oder Allyl und unter Alkinyl Ethinyl oder Propin-1-yl-1 zu verstehen.

Als Cycloalkylreste einer Cycloalkyloxi- bzw. einer Cycloalkylmethoxi-Gruppe kommen je nach Zahl der genannten Kohlenstoffatome Cyclopropyl, Cyclobutyl, Cyclopentyl oder Cyclohexyl in Frage.

Unter dem Dioxoanyl-Rest ist insbesondere die 1,3-Dioxolan-yl-1-Gruppe zu verstehen, die in 4- und/oder in 5-Stellung durch eine Methyl-, Ethyl- oder Propylgruppe substituiert sein kann. Azol-1-yl steht für Pyrazol-1-yl oder 1,2,4-Triazol-1-yl.

In den Verbindungen der Formel I können die Substituenten $R^1$ zum Stickstoffatom in cis-, trans- und cis/trans-Stellung stehen. Die Verbindungen der Formel I können dafür in sterisch reiner Form oder als Isomerengemische vorliegen.

Die Verbindungen der Formel I können hergestellt werden, indem man

a) ein N-substituiertes Cyclohexylamin der Formel II

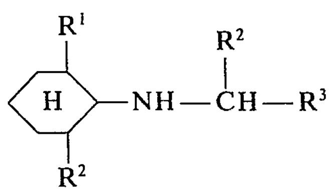

(II)

worin $R^1$, $R^2$ und $R^3$ die oben angegebene Bedeutung besitzen, mit Chloressigsäureanhydrid oder einem Chloressigsäurehalogenid umsetzt oder

b) – falls $R^2$ Wasserstoff und $R^3$ $C_{1-8}$-Alkyloxi, $C_{3-6}$-Alkenyloxi, $C_{3-6}$-Alkinyloxi, $C_{3-6}$-Cycloalkylmethoxi, $C_{4-6}$-Cycloalkyloxi, $C_{1-6}$-Alkyloxiethoxi, Tetrahydrofuranylmethoxi, Tetrahydropyranylmethoxi oder Azol-1-yl bedeuten – ein N-Chlormethylchloressigsäurecyclohexylamid der Formel III

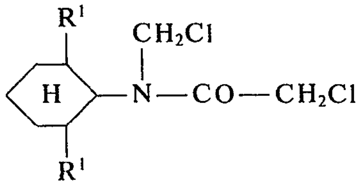

(III)

worin $R^1$ die oben angegebene Bedeutung hat,

mit einer Verbindung der Formel $R^3H$, worin $R^3$ die oben unter b) angegebene Bedeutung besitzt, umsetzt.

Gemäß a) werden die Verbindungen II mit Chloressigsäureanhydrid oder vorzugsweise mit Chloracetylchlorid, vorzugsweise in einem gegenüber den Reaktanden inerten Lösungsmittel, zu den erfindungsgemäßen Chloressigsäurecyclohexylamiden umgesetzt. Dabei können organische oder anorganische Basen wie Trialkylamine, Pyridine, Hydrogencarbonate, Carbonate oder Alkalihydroxide verwendet werden. Als Verdünnungsmittel kommen vorzugsweise mit Wasser nicht mischbare Solventien wie Halogenkohlenwasserstoffe, z.B. Methylenchlorid, Chloroform, Chlorbenzol, oder Kohlenwasserstoffe, z.B. Cyclohexan, Heptan, Toluol, Xylol, in Frage. Chloracetylchlorid wird in stöchiometrischer Menge und die Base in mindestens stöchiometrischer Menge, bezogen auf eingesetztes N-substituiertes Cyclohexylamin der Formel II, verwendet. Die Umsetzungen mit Chloracetylchlorid werden bei −20 bis +150°C, vorzugsweise bei 0 bis 110°C durchgeführt.

Die Ausgangsverbindungen der Formel II können hergestellt werden durch Umsetzungen eines Cyclohexylamins der Formel

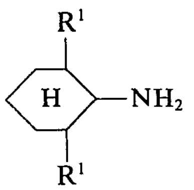

(IV)

in der $R^1$ die angegebene Bedeutung hat, mit einem Alkylierungsmittel der Formel

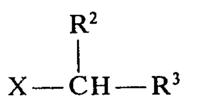

(V)

in welcher $R^2$ und $R^3$ die oben angegebene Bedeutung haben und X für eine Abgangsgruppe, wie z.B. Chlorid, Bromid, Iodid, p-Toluolsulfonat, Benzolsulfonat, Trifluoracetat, steht. Die Alkylierungsreaktion kann je nach Art und Reaktivität des Alkylierungsmittels ohne Solvens durchgeführt werden. Als Verdünnungsmittel eignen sich aprotisch dipolare Lösungsmittel wie Nitrile, z.B. Acetonitril, Amide wie Dimethylformamid, aber auch protische Lösungsmittel wie Alkohole, z.B. Ethanol, Isopropanol und Wasser und Gemische dieser Lösungsmittel. Zur Bindung des gebildeten HX, wie z.B. Chlorwasserstoff oder Bromwasserstoff, können anorganische Basen wie Carbonate, Hydrogencarbonat, Alkalihydroxide und organische Basen wie Trialkylamine und ebenso das als Reaktionspartner benutzte Cyclohexylamin der Formel II verwendet werden. Zweckmäßigerweise wird je Mol Alkylierungsmittel der Formel III mindestens 1 Mol Cyclohexylamin der Formel II und mindestens 1 Mol anorganische oder organische Base eingesetzt. Die Reaktionstemperaturen liegen bei 25 bis 150°C. Die N-substituierten Cyclohexylamine der Formel IV werden gegebenenfalls nach Freisetzung aus ihren Salzen und gegebenenfalls Entfernen von mit Wasser mischbaren Lösungsmitteln meist durch fraktionierte Destillation isoliert.

Ein Teil der N-substituierten Cyclohexylamide der Formel II, in der $R^3$ weder über ein Sauerstoffatom noch über ein Stickstoffatom mit der CH-Gruppe verknüpft ist, kann auch dadurch hergestellt werden, daß man ein Cyclohexylamin der Formel IV mit einer Carbonylverbindung der Formel

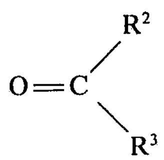

(VI)

in welcher $R^2$ die angegebene Bedeutung hat und $R^3$ nicht über ein O-Atom oder ein N-Atom mit der Carbonylgruppe verbunden ist, unter Abspaltung von Wasser umsetzt und die gebildete Schiff'sche Base mit katalytisch angeregtem Wasserstoff oder einem Wasserstoffüberträger wie komplexen Hydriden, z.B. $NaBH_4$ oder $LiAlH_4$, zu einem N-substituierten Cyclohexylamin der Formel II reduziert und dieses in der oben beschriebenen Weise mit Chloracetylchlorid umsetzt.

Das bei der Herstellung der Schiff'schen Base gebildete Reaktionswasser wird zweckmäßigerweise durch azeotrope Destillation entfernt mit den üblichen Schleppmitteln, z. B. Kohlenwasserstoffe wie n-Pentan, n-Hexan, Cyclohexan, Benzol, Toluol, Xylol, Methylenchlorid oder Chloroform.

Als Katalysatoren für die Hydrierungen der Schiff'schen Basen eignen sich Edelmetallkatalysatoren wie Palladium oder Platin auf geeigneten Trägern sowie Raney-Nickel bei Temperaturen zwischen 20 bis 200°C, vorzugsweise bei 50 bis 150°C, und Wasserstoffdrucken zwischen 10 bis 500 bar.

Die noch nicht beschriebenen Verbindungen der Formel IV lassen sich in besonders einfacher Weise und in sterisch einheitlicher Form durch hydrierende Aminierung von 2,6-Dialkylphenolen und anschließende destillative Trennung herstellen.

Die Reaktion b) wird in Gegenwart organischer oder anorganischer Basen wie Trialkylaminen, Pyridinen, Hydrogencarbonaten, Alkalicarbonaten oder Alkalihydroxiden bei 20 bis 120°C durchgeführt.

Die Verbindungen der Formel III sind durch Umsetzung von einem Cyclohexylamin der Formel II mit Formaldehyd zu einem Formimin der Formel

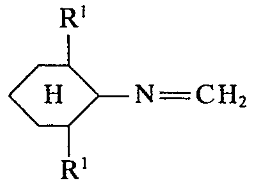

(VII)

in welchem $R^1$ die oben angegebene Bedeutung hat, und anschließender Addition von Chloracetylchlorid an das Formimin zugänglich.

Zur Herstellung der Schiff'schen Base der Formel VII wird 1 Mol Cyclohexylamin der Formel IV mit einer wäßrigen Lösung mit mindestens molarer Menge an Formaldehyd gegebenenfalls unter Verwendung eines Verdünnungsmittels, wie z. B. Kohlenwasserstoffe oder Halogenkohlenwasserstoffe bei –5 bis 100°C, vorzugsweise 0 bis 50°C, insbesondere Raumtemperatur, umgesetzt. Die Schiff'sche Base wird anschließend getrocknet und gegebenenfalls nach Destillation an mindestens eine molare Menge Chloracetylchlorid bei 0 bis 100°C, gegebenenfalls in Gegenwart eines Verdünnungsmittels, das gegenüber den Reaktionspartnern inert ist, zur N-Chlormethylchloressigsäurecyclohexylamid der Formel III addiert. Dieses Zwischenprodukt kann bei stöchiometrischem Arbeiten als Öl isoliert werden. Einfacher ist es jedoch, das Rohprodukt in der Reaktionslösung mit einem Alkohol oder Glykolmonoether oder Pyrazol bzw. 1,2,4-Triazol unter Verwendung einer Base umzusetzen. Im allgemeinen werden, bezogen auf 1 Mol eingesetzte Schiff'sche Base, 1 bis 10 Mol Alkohol, vorzugsweise 2 bis 5 Mol Alkohol, bzw. 1 bis 2 Mol Pyrazol bzw. Triazol eingesetzt und zum Binden des freiwerdenden Chlorwasserstoffs mindestens 1 Mol Base, vorzugsweise tertiäre Amine, z. B. Triethylamin, verwendet. Dieser Reaktionsschritt wird bei Temperaturen von 0 bis 100°C, vorzugsweise bei Raumtemperatur, durchgeführt. Zur Isolierung der erfindungsgemäßen Chloressigsäurecyclohexylamide der Formel I werden Überschuß an Alkohol bzw. Azol und gebildetes Aminhydrochlorid durch Waschen mit Wasser entfernt und nach Trocknen und Verdampfen des Lösungsmittels das Rohprodukt gegebenenfalls durch Destillation oder Umkristallisation weiter gereinigt.

Die im folgenden aufgeführten Beispiele beschreiben die verschiedenen Herstellungsmöglichkeiten der erfindungsgemäßen Chloressigsäurecyclohexylamide. In den Beispielen verhalten sich Gewichtsteile zu Volumenteile wie Kilogramm zu Liter.

## Beispiel 1

### a) Herstellung des Ausgangsmaterials

In einen Rührautoklaven mit einem Inhalt von 10 000 Raumteilen werden 1650 Gewichtsteile 2,6-Dimethylphenol und 150 Gewichtsteile eines pulverförmigen Katalysators, der 10,0 Gew.-% Palladium, 5,0 Gew.-% Praseodymoxid auf Aluminiumoxid enthält, eingefüllt. Der Autoklav wird verschlossen und es werden 1370 Gewichtsteile Ammoniak aufgepreßt. Nun wird auf 250° erhitzt und durch Zufuhr von Wasserstoff ein Druck von 300 bar eingestellt. Man hält so lange auf Reaktionstemperatur, bis konstanter Druck erreicht ist (ca. 10 Stunden). Anschließend läßt man abkühlen, filtriert und erhält 1691 Gewichtsteile (98,5 %) 2,6-Dimethylcyclohexylamin, die nach gaschromatographischer und NMR-Analyse folgende Isomerenverteilung haben:

56 % trans,trans-2,6-Dimethylcyclohexylamin
14 % cis,cis-2,6-Dimethylcyclohexylamin
30 % cis,trans-2,6-Dimethylcyclohexylamin.

Die Trennung der Isomeren gelingt durch fraktionierte Destillation an einer Kolonne mit 45 Trennstufen. Die einzelnen isomeren Formen sind in über 95 %iger Reinheit bei folgenden Siedetemperaturen erhalten worden:

trans-trans-2,6-Dimethylcyclohexylamin, Kp/1012 mbar = 166°C
cis,cis-2,6-Dimethylcyclohexylamin, Kp/1012 mbar = 168°C
cis,trans-2,6-Diemthylcyclohexylamin, Kp/1012 mbar = 172°C.

Zu einer Lösung von 63,5 Gewichtsteilen trans,trans-2,6-Dimethylcyclohexylamin in 150 Volumenteilen Acetonitril wurde eine Mischung aus 29,8 Gewichtsteilen Propargylbromid und 20 Volumenteilen Acetonitril unter Stickstoff und Rühren zugetropft bei 20 bis 25°C, nachgerührt und 5 h unter Rückfluß erhitzt. Nach Verdampfen des Acetonitrils wurde der Rückstand zwischen 100 Volumenteilen Methylenchlorid und 150 Volumenteilen einer 10 %igen Kaliumhydroxidlösung verteilt, die organische Phase abgetrennt und dreimal mit je 100 Volumenteilen Methylenchlorid extrahiert. Nach dem Verdampfen des Methylenchlorids aus den vereinigten organischen Phasen erhielt man durch fraktionierte Destillation im Vakuum unter Stickstoff 28,0 Gewichtsteile N-(Propargyl)-trans,trans-2,6-dimethylcyclohexylamin, Sdp.: 93 bis 96°C/18 mbar ($n_D^{20}$ = 1,4717), und einen Vorlauf von 15,0 Gewichtsteilen, Kp. 63 bis 77°C/16 mbar, der 25 % des gewünschten Produkts enthielt.

b) Herstellung des Endprodukts

Zu einer Mischung von 25,7 Gewichtsteilen N-(Propargyl)-trans,trans-2,6-dimethylcyclohexylamin in 150 Volumenteilen Toluol und 81,2 Gewichtsteilen 10 %ige Natronlauge wurden bei 0°C unter intensiver Vermischung 19,4 Gewichtsteile Chloracetylchlorid in Vermischung 19,4 Gewichtsteile Chloracetylchlorid in 20 Volumenteilen Toluol in 30 Minuten zugetropft und 3 h bei 20°C nachgerührt. Aus der Toluolphase isolierte man nach Waschen mit Wasser, Trocknen und Verdampfen durch Destillation des Rückstands 22,7 Gewichtsteile N-(Propargyl)-N-(trans,trans-2,6-dimethylcyclohexyl)-$\alpha$-chloracetamid, Kp. 132 bis 134°C/0,4 mbar (Fp. 59 bis 62°C).

Beispiel 2

a) Herstellung des Ausgangsmaterials

Zu einer Lösung von 76,2 Gewichtsteilen trans,trans-2,6-Dimethylcyclohexylamin in 200 Volumenteilen n-Hexan wurden unter Stickstoff und Rühren und Rückfluß 52,8 Gewichtsteile Methoxyaceton in 1 h zugetropft und gleichzeitig 10,5 Volumenteile Wasser durch azeotrope Destillation in etwa 3 h entfernt. Nach dem Verdampfen des n-Hexans isolierte man durch Destillation des Rückstandes unter Stickstoff 83,0 Gewichtsteile Methoxy-isopropyliden-trans,trans-2,6-dimethylcyclohexylamin, Kp. 107 bis 109°C/17 mbar ($n_D^{20}$ = 1,4638).

In eine Lösung von 76,0 Gewichtsteilen Methoxy-isopropyliden-trans,trans-2,6-dimethylcyclohexylamin in 250 Volumenteilen Methanol wurden bei 10 bis 25°C 29,7 Gewichtsteile $NaBH_4$ in 2 h unter Rühren eingetragen (Eiskühlung) und 1,5 h unter Rückfluß erhitzt. Nach dem Verdampfen des Methanols und Verteilen des Rückstandes zwischen Methylenchlorid und Wasser isolierte man durch Destillation der organischen Phase 64,0 Gewichtsteile N-(1-Methoxy-prop-2-yl)-trans,trans-2,6-dimethylcyclohexylamin, Kp. 117 bis 118°C/22 mbar.

b) Herstellung des Endprodukts

Zu einer Lösung von 19,9 Gewichtsteilen N-(1-Methoxy-prop-2-yl)-trans,trans-2,6-dimethylcyclohexylamin in 100 Volumenteilen Toluol wurde unter Stickstoff bei 80°C eine Lösung von 12,4 Gewichtsteilen Chloracetylchlorid in 20 Volumenteilen Toluol in 15 Minuten zugetropft. Anschließend wurde 3 h unter Rückfluß und unter Durchleiten von Stickstoff erhitzt. Nach dem Abkühlen wurde nacheinander mit 200 Volumenteilen Wasser, 50 Volumenteilen 2 N HCl, 50 Volumenteilen $NaHCO_3$-Lösung und 50 Volumenteilen Wasser gewaschen und getrocknet. Nach Verdampfen des Toluols isolierte man 20,5 Gewichtsteile N-(1-Methoxy-prop-2-yl)-N-trans,trans-2,6-dimethylcyclohexyl)-$\alpha$-chloracetamid, Fp. 120 bis 122°C (Methanol).

Beispiel 3

a) Herstellung des Ausgangsmaterials

Zu einer Lösung von 152,0 Gewichtsteilen trans,trans-2,6-dimethylcyclohexylamin in 450 Volumenteilen Methylenchlorid wurden bei 25 bis 28°C unter Stickstoff und unter Rühren 110,0 Gewichtsteile einer 37 %igen Formalin-Lösung in 30 min zugetropft und 4 h nachgerührt. Die organische Phase wurde abgetrennt und zweimal mit je 100 Volumenteilen Wasser gewaschen und getrocknet. Nach dem Verdampfen des Methylenchlorids isolierte man durch Destillation unter Stickstoff 136,0 Gewichtsteile trans,trans-2,6-Dimethylcyclohexylformimin, Kp. 59 bis 60°C/20 mbar ($n_D^{20}$ = 1,4634).

b) Herstellung des Endprodukts

Zu einer Lösung von 124,3 Gewichtsteilen Chloracetylchlorid in 100 Volumenteilen Cyclohexan wurde unter Eiskühlung und Rühren sowie unter Ausschluß von Feuchtigkeit eine Lösung von 152,9 Gewichtsteilen trans,trans-2,6-Dimethylcyclohexylformimin in 100 Volumenteilen Cyclohexan bei 20 bis 25°C zugetropft und 16 h bei 25°C nachgerührt.

Zu dieser Mischung wurde unter Verdünnen mit 200 Volumenteilen Cyclohexan eine Mischung aus 184,0 Gewichtsteilen Ethanol und 112,0 Gewichtsteilen Triethylamin unter Eiskühlung und Rühren bei 20 bis 25°C zugetropft und 24 h bei Raumtemperatur nachgerührt. Nach dreimaligem Waschen des Ansatzes mit je 150 Volumenteilen Wasser, Trocknen und Verdampfen des Cyclohexans isolierte man 295,0 Gewichtsteile Öl, aus dem durch Destillation unter Stickstoff 262,0 Gewichtsteile N-(Ethoxymethyl)-N-(trans,trans-2,6-dimethylcyclohexyl)-$\alpha$-chloracetamid, Kp. 122 bis 124°C/0,5 mbar ($n_D^{20}$ = 1,4855), erhalten wurden.

Analog Beispiel 1 a), 2 a) und 3 a) lassen sich die in den folgenden Tabellen aufgeführten Ausgangsverbindungen herstellen bzw. wurden hergestellt:

Tabelle 1

Verbindungen der Formel II, $R^1$ = Methylgruppen in trans,trans-Stellung zum N-Atom

| $R^2$ | $R^3$ | Kp (°C)/mbar | $n_D^{20}$ |
|---|---|---|---|
| H | $CH{=}CH_2$ | 77–82/14 | 1,4627 |
| H | $C(CH_3){=}CH_2$ | 96–99/16 | 1,4656 |
| H | $CH{=}CH(CH_3)$ | | |
| H | $C{\equiv}CH$ | 93–96/18 | 1,472 |
| $CH_3$ | $C{\equiv}CH$ | | |
| H | $CH_2OCH_3$ | 117–120/20 | |
| H | $CH_2OC_2H_5$ | | |
| $CH_3$ | $CH_2OCH_3$ | 117–118/22 | |
| $CH_3$ | $CH_2OC_2H_5$ | | |
| H | $CO_2CH_3$ | 132–134/28 | |
| H | $CO_2C_2H_5$ | 124–126/12 | 1,4586 |
| $CH_3$ | $CO_2CH_3$ | 84–91/0,4 | 1,4683 |
| H | $\alpha$-Furyl | 82–83/0,2 | |

Fortsetzung

| $R^2$ | $R^3$ | Kp (°C)/mbar | $n_D^{20}$ |
|---|---|---|---|
| H | α-Tetrahydrofuryl | 144–147/18 | 1,4752 |
| H | $CH(OCH_3)_2$ | | |
| H | $CH(OC_2H_5)_2$ | 146–149/24 | |
| $CH_3$ | $CH(OCH_3)_2$ | | |
| H | $CH(CH_3)OCH_3$ | 110–112/24 | |
| H | $C_6H_5$ | Öl | |

Tabelle 2

Verbindungen der Formel II, $R^1$ = Methylgruppen in cis,trans-Stellung zum N-Atom

| $R^2$ | $R^3$ | Kp (°C)/mbar | $n_D^{20}$ |
|---|---|---|---|
| H | $CH{=}CH_2$ | | |
| H | $C(CH_3){=}CH_2$ | | |
| H | $C{=}CH(CH_3)$ | | |
| H | $C{\equiv}CH$ | 96–99/22 | |
| $CH_3$ | $C{\equiv}CH$ | | |
| H | $CH_2OCH_3$ | | |
| H | $CH_2OC_2H_5$ | | |
| $CH_3$ | $CH_2OCH_3$ | | |
| $CH_3$ | $CH_2OC_2H_5$ | | |
| H | $CO_2CH_3$ | | |
| H | $CO_2C_2H_5$ | 136–138/22 | |
| $CH_3$ | $CO_2CH_3$ | | |
| H | α-Furyl | | |
| H | α-Tetrahydrofuryl | | |
| H | $CH(OCH_3)_2$ | | |
| H | $CH(OC_2H_5)_2$ | | |
| $CH_3$ | $CH(OCH_3)_2$ | | |
| H | $CH(CH_3)OCH_3$ | | |
| H | $C_6H_5$ | | |

Tabelle 3

Verbindungen der Formel II, $R^1$ = Methylgruppen in cis,cis-Stellung zum N-Atom

| $R^2$ | $R^3$ | Kp (°C)/mbar | $n_D^{20}$ |
|---|---|---|---|
| H | $CH{=}CH_2$ | | |
| H | $C(CH_3){=}CH_2$ | | |
| H | $C{=}CH(CH_3)$ | | |
| H | $C{\equiv}CH$ | 94–98/24 | |
| $CH_3$ | $C{\equiv}CH$ | | |
| H | $CH_2OCH_3$ | | |
| H | $CH_2OC_2H_5$ | | |
| $CH_3$ | $CH_2OCH_3$ | | |
| $CH_3$ | $CH_2OC_2H_5$ | | |
| H | $CO_2CH_3$ | | |
| H | $CO_2C_2H_5$ | 134–136/20 | |
| $CH_3$ | $CO_2CH_3$ | | |
| H | α-Furyl | | |
| H | α-Tetrahydrofuryl | | |
| H | $CH(OCH_3)_2$ | | |
| H | $CH(OC_2H_5)_2$ | | |
| $CH_3$ | $CH(OCH_3)_2$ | | |
| H | $CH(CH_3)OCH_3$ | | |
| H | $C_6H_5$ | | |

Tabelle 4

2,6-Dialkylcyclohexylformimine der Formel

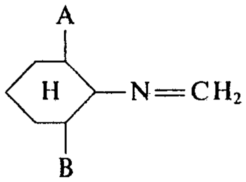

| A | B | Kp (°C)/mbar | $n_D^{20}$ |
|---|---|---|---|
| cis-$CH_3$ | cis-$CH_3$ | 57–59/20 | 1,4605 |
| cis-$CH_3$ | trans-$CH_3$ | 62–64/22 | 1,4618 |
| trans-$CH_3$ | trans-$CH_3$ | 59–60/20 | 1,4638 |
| cis-$C_2H_5$ | cis-$C_2H_5$ | 92–94/20 | |
| cis-$C_2H_5$ | trans-$C_2H_5$ | 110/40 | |
| trans-$C_2H_5$ | trans-$C_2H_5$ | | |

Analog Beispiel 1 b), 2 b) und 3 b) lassen sich die in den folgenden Tabellen aufgeführten Verbindungen herstellen bzw. wurden hergestellt:

Tabelle 5

Verbindungen der Formel I, $R^1$ = Methylgruppen in trans,trans-Stellung zum N-Atom

| Nr. | $R^2$ | $R^3$ | Kp; $n_D^{20}$; Fp |
|---|---|---|---|
| 9 | H | $CH{=}CH_2$ | 125/0,3 |
| 10 | $CH_3$ | $CH{=}CH_2$ | |
| 11 | H | $(C(CH_3){=}CH_2$ | 130–132/0,5 |
| 12 | H | $CH{=}CH(CH_3)$ | |
| 13 | H | $CH_2C{\equiv}CH$ | |
| 14 | $CH_3$ | $C{\equiv}CH$ | |
| 15 | H | $C{\equiv}C{-}CH_3$ | |
| 16 | H | $CH_2OCH_3$ | 140–145/0,2 |
| 17 | H | $CH_2OC_2H_5$ | |
| 18 | H | $CH_2OnC_3H_7$ | |
| 19 | $CH_3$ | $CH_2OCH_3$ | 123–125 |
| 20 | $CH_3$ | $CH_2OC_2H_5$ | |

Fortsetzung

| Nr. | $R^2$ | $R^3$ | Kp; $n_D^{20}$; Fp |
|---|---|---|---|
| 21 | H | $CO_2CH_3$ | 164–166/0,5 |
| 22 | $CH_3$ | $CO_2CH_3$ | 165–170/0,4 |
| 23 | H | $CO_2C_2H_5$ | 75–77 |
| 24 | H | $CO_2nC_3H_7$ | |
| 25 | H | $CO_2iC_3H_7$ | |
| 26 | H | α-Furyl | 165–166/0,7; 1,5712 |
| 27<br>28 | H | β-Furyl | |
| 29 | H | α-Tetrahydrofuryl | 166–168/0,4 |
| 30 | $CH_3$ | α-Furyl | |
| 31 | H | $CH(OCH_3)_2$ | |
| 32 | H | $CH(OC_2H_5)_2$ | |
| 33 | $CH_3$ | $CH(OCH_3)_2$ | |
| 34 | H | 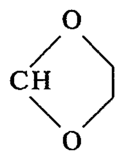 | |
| 35 | H | 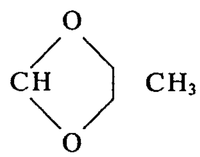 | |
| 36 | H | 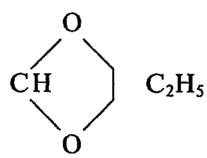 | |
| 37 | $CH_3$ | 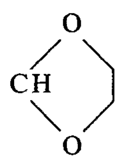 | |
| 38 | H | $OCH_3$ | 121–123/0,4 |
| 39 | H | $OnC_5H_{11}$ | |
| 40 | H | $OnC_3H_7$ | 135/0,05 |
| 41 | H | $OiC_3H_7$ | 133–135/0,4 |

Fortsetzung

| Nr. | $R^2$ | $R^3$ | Kp; $n_D^{20}$; Fp |
|---|---|---|---|
| 42 | H | $OnC_4H_9$ | 140–142/0,08 |
| 43 | H | Osec. $C_4H_9$ | 136–138/0,5; 1,4827 |
| 44 | H | $OiC_4H_9$ | 138–140/0,5; 1,481 |
| 45 | H | Otert. $C_4H_9$ | 102–103 |
| 46 | H | O—Cyclopentyl | 40–42 |
| 47 | H | O—Cyclohexyl | |
| 48 | H | $O-CH_2-$ 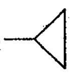 | 148–150; 1,4956 |
| 49 | H | $O-CH_2-$ 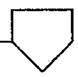 | |
| 50 | H | $OCH_2CH=CH_2$ | 135–137/0,5; 1,4955 |
| 51 | H | $OCH_2C(CH_3)=CH_2$ | 135/0,5; 1,495 |
| 52 | H | $OCH_2CH=C(CH_3)_2$ | 160–163/0,4 |
| 53 | H | $OCH_2C\equiv CH$ | 145–147/0,4; 1,502 |
| 54 | H | $OCH_2C\equiv C-CH_3$ | |
| 55 | H | $O(CH_2)_2OCH_3$ | 148–150/0,3 |
| 56 | H | $O(CH_2)_2OC_2H_5$ | 150–152/0,3 |
| 57 | H | $O(CH_2)_2OnC_3H_7$ | 161–163/0,3 |
| 58 | H | $O(CH_2)_2OnC_4H_9$ | 168–170/0,2 |
| 59 | H | Pyrazol-1 | Öl |
| 60 | H | 1,2,4-Triazolyl-1 | |
| 61 | H | $CH(CH_3)OCH_3$ | 146–148/0,3 |
| 62 | H | $C_6H_5$ | 175–178/0,2 |
| 63 | H | $OCH_2-$ 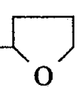 | 162/2,0 |

Tabelle 6

Verbindungen der Formel I, $R^1$ = Methylgruppen in cis,trans-Stellung zum N-Atom

| Nr. | $R^2$ | $R^3$ | Kp; $n_D^{20}$; Fp |
|---|---|---|---|
| 69 | H | $CH{=}CH_2$ | |
| 70 | $CH_3$ | $CH{=}CH_2$ | |
| 71 | H | $(C(CH_3){=}CH_2$ | |
| 72 | H | $CH{=}CH(CH_3)$ | |
| 73 | H | $C{\equiv}CH$ | 143−145/0,7 |
| 74 | $CH_3$ | $C{\equiv}CH$ | |
| 75 | H | $C{\equiv}C{-}CH_3$ | |
| 76 | H | $CH_2OCH_3$ | |
| 77 | H | $CH_2OC_2H_5$ | |
| 78 | H | $CH_2OnC_3H_7$ | |
| 79 | $CH_3$ | $CH_2OCH_3$ | |
| 80 | $CH_3$ | $CH_2OC_2H_5$ | |
| 81 | H | $CO_2CH_3$ | |
| 82 | $CH_3$ | $CO_2CH_3$ | |
| 83 | H | $CO_2C_2H_5$ | 160−162/0,3 |
| 84 | H | $CO_2nC_3H_7$ | |
| 85 | H | $CO_2iC_3H_7$ | |
| 86 | H | α-Furyl | |
| 87 | H | β-Furyl | |
| 88 | H | α-Tetrahydrofuryl | |
| 89 | $CH_3$ | α-Furyl | |
| 90 | H | $CH(OCH_3)_2$ | |
| 91 | H | $CH(OC_2H_5)_2$ | |
| 92 | $CH_3$ | $CH(OCH_3)_2$ | |
| 93 | H | 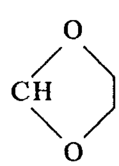 | |

Fortsetzung

| Nr. | $R^2$ | $R^3$ | Kp; $n_D^{20}$; Fp |
|---|---|---|---|
| 94 | H | 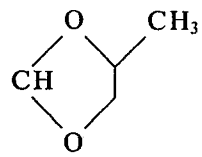 | |
| 95 | H | 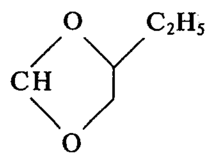 | |
| 96 | $CH_3$ | 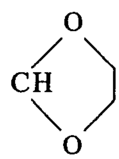 | |
| 97 | H | $OCH_3$ | 50–53 |
| 98 | H | $OC_2H_5$ | 63–65 |
| 99 | H | $OnC_3H_7$ | 140/0,5 |
| 100 | H | $OiC_3H_7$ | |
| 101 | H | $OnC_4H_9$ | |
| 102 | H | $Osec.C_4H_9$ | |
| 103 | H | $OiC_4H_0$ | |
| 104 | H | $Otert.C_4H_9$ | |
| 105 | H | O—Cyclopentyl | |
| 106 | H | O—Cyclohexyl | |
| 107 | H | $OCH_2$— 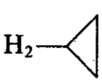 | |
| 108 | H | $OCH_2$— 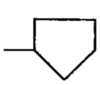 | |
| 109 | H | $OCH_2CH{=}CH_2$ | |
| 110 | H | $OCH_2C(CH_3){=}CH_2$ | |
| 111 | H | $OCH_2CH{=}C(CH_3)_2$ | |
| 112 | H | $OCH_2C{\equiv}CH$ | |
| 113 | H | $OCH_2C{\equiv}C{-}CH_3$ | |
| 114 | H | $O(CH_2)_2OCH_3$ | |

Fortsetzung

| Nr. | $R^2$ | $R^3$ | Kp; $n_D^{20}$; Fp |
|---|---|---|---|
| 115 | H | $O(CH_2)_2OC_2H_5$ | |
| 116 | H | $O(CH_2)_2OnC_3H_7$ | |
| 117 | H | $O(CH_2)_2OnC_4H_9$ | |
| 118 | H | 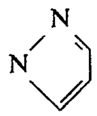 | Öl |
| 119 | H | 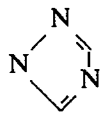 | 103–106 |
| 120 | H | $(CH(CH_3)OCH_3$ | |
| 121 | H | $C_6H_5$ | |

Tabelle 7

Verbindungen der Formel I, $R^1$ = Methylgruppen in cis,cis-Stellung zum N-Atom

| Nr. | $R^2$ | $R^3$ | Kp; $n_D^{20}$; Fp |
|---|---|---|---|
| 127 | H | $CH{=}CH_2$ | |
| 128 | $CH_3$ | $CH{=}CH_2$ | |
| 129 | H | $C(CH_3){=}CH_2$ | |
| 130 | H | $CH{=}CH(CH_3)$ | |
| 131 | H | $C{\equiv}CH$ | 142–146/0,5 |
| 132 | $CH_3$ | $C{\equiv}CH$ | |
| 133 | H | $C{\equiv}C{-}CH_3$ | |
| 134 | H | $CH_2OCH_3$ | |
| 135 | H | $CH_2OC_2H_5$ | |
| 136 | H | $CH_2OnC_3H_7$ | |
| 137 | $CH_3$ | $CH_2OCH_3$ | |
| 138 | $CH_3$ | $CH_2OC_2H_5$ | |
| 139 | H | $CO_2CH_3$ | |
| 140 | $CH_3$ | $CO_2CH_3$ | |
| 141 | H | $CO_2C_2H_5$ | |

Fortsetzung

| Nr. | $R^2$ | $R^3$ | Kp; $n_D^{20}$; Fp |
|---|---|---|---|
| 142 | H | $CO_2nC_3H_7$ | |
| 143 | H | $CO_2iC_3H_7$ | |
| 144 | H | α-Furyl | |
| 145<br>146 | H | β-Furyl | |
| 147 | H | α-Tetrahydrofuryl | |
| 148 | $CH_3$ | α-Furyl | |
| 149 | H | $CH(OCH_3)_2$ | |
| 150 | H | $CH(OC_2H_5)_2$ | |
| 151 | $CH_3$ | $CH(OCH_3)_2$ | |
| 152 | H | 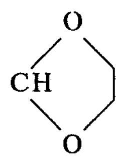 | |
| 153 | H | 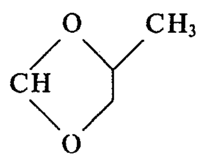 | |
| 154 | H | 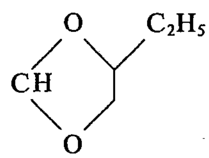 | |
| 155 | $CH_3$ | 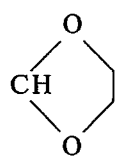 | |
| 156 | H | $OCH_3$ | 78–81 |
| 157 | H | $OC_2H_5$ | 126–128/0,4 |
| 158 | H | $OnC_3H_7$ | 133–135/0,4 |
| 159 | H | $OiC_3H_7$ | 130–132/0,5 |
| 160 | H | $OnC_4H_9$ | 140–143/0,3 |
| 161 | H | Osec.$C_4H_9$ | 137–139/0,5 |
| 162 | H | $OiC_4H_9$ | 138–140/0,5 |
| 163 | H | Otert.$C_4H_9$ | |

Fortsetzung

| Nr. | $R^2$ | $R^3$ | Kp; $n_D^{20}$; Fp |
|---|---|---|---|
| 164 | H | O—Cyclopentyl | |
| 165 | H | O—Cyclohexyl | |
| 166 | H | $OCH_2$— 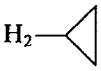 | |
| 167 | H | $OCH_2$— 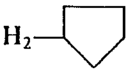 | |
| 168 | H | $OCH_2CH{=}CH_2$ | |
| 169 | H | $OCH_2C(CH_3){=}CH_2$ | |
| 170 | H | $OCH_2CH{=}C(CH_3)_2$ | |
| 171 | H | $OCH_2C{\equiv}CH$ | |
| 172 | H | $OCH_2C{\equiv}C{-}CH_3$ | |
| 173 | H | $O(CH_2)_2OCH_3$ | |
| 174 | H | $O(CH_2)_2OC_2H_5$ | |
| 175 | H | $O(CH_2)_2OnC_3H_7$ | |
| 176 | H | $O(CH_2)_2OnC_4H_9$ | |
| 177 | H | 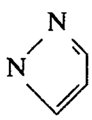 | |
| 178 | H | 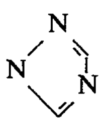 | |
| 179 | H | $CH(CH_3)OCH_3$ | |
| 180 | H | $C_6H_5$ | |

Tabelle 8

Verbindungen der Formel I, $R^1$ = Ethylgruppen in trans,trans-Stellung zum N-Atom

| Nr. | $R^2$ | $R^3$ | Kp; $n_D^{20}$; Fp |
|---|---|---|---|
| 181 | H | $OCH_3$ | 132/0,3 |
| 182 | H | $OC_2H_5$ | 146/0,6 |
| 183 | H | O—n-$C_3H_7$ | 154/0,5 |

Fortsetzung

| Nr. | $R^2$ | $R^3$ | Kp; $n_D^{20}$; Fp |
|---|---|---|---|
| 184 | H | $O—n-C_4H_9$ | |
| 185 | H | $O—i-C_4H_9$ | |
| 186 | H | $O—sec.-C_4H_9$ | |

Tabelle 9

Verbindungen der Formel I, $R^1$ = Ethylgruppen in cis,trans-Stellung zum N-Atom

| Nr. | $R^2$ | $R^3$ | Kp; $n_D^{20}$; Fp |
|---|---|---|---|
| 187 | H | $OCH_3$ | 136–138/0,3 |
| 188 | H | $OC_2H_5$ | 138–140/0,2 |
| 189 | H | $O—n-C_3H_7$ | 148–150/0,2 |
| 190 | H | $O—n-C_4H_9$ | 160–162/0,5 |
| 191 | H | $O—i-C_4H_9$ | |
| 192 | H | $O—sec.-C_4H_9$ | |

Tabelle 10

Verbindungen der Formel I, $R^1$ = Ethylgruppen in cis,cis-Stellung zum N-Atom

| Nr. | $R^2$ | $R^3$ | Kp; $n_D^{20}$; Fp |
|---|---|---|---|
| 193 | H | $OCH_3$ | 54–56 |
| 194 | H | $OC_2H_5$ | 142–144/0,3; 1,4906 |
| 195 | H | $O—n-C_3H_7$ | 152–154/0,4; 1,4898 |
| 196 | H | $O—n-C_4H_9$ | 160–162/0,5; 1,4875 |
| 197 | H | $O—i-C_4H_9$ | |
| 198 | H | $O—sec.-C_4H_9$ | |

Die neuen Wirkstoffe besitzen insbesondere eine starke herbizide Aktivität und eine gute Verträglichkeit gegen Kulturpflanzen.

Ihre Anwendung erfolgt z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen. Die Anwendungsformen richten sich ganz nach den Verwendungszwecken.

Zur Herstellung von direkt versprühbaren Lösungen, Emulsionen, Pasten und Öldispersionen kommen

Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, z. B. Dimethylformamid, Dimethylsulfoxid, N-Methylpyrrolidon und Wasser in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen:

Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol-, Octylphenol-, Nonylphenol, Alkylphenylpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischer oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Silicagel, Kieselsäuren, Kieselgele, Silikate, Talkum, Kaolin, Attaclay, Kalkstein, Kalk, Kreide, Talum, Bolus, Löß, Ton, Dolomit, Diatomeenerde, Calcium- und Magnesiumsulfat, Magnesiumoxid und gemahlene Kunststoffe.

Beispiele für Formulierungen sind:

I 20 Gewichtsteile der Verbindung des Beispiels 39 werden in 3 Gewichtsteilen des Natriumsalzes der Diisobutylnaphthalin-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.

II 3 Gewichtsteile der Verbindung des Beispiels 40 werden mit 97 Gewichtsteilen feinteiligem Kaolin innig vermischt. Man erhält auf diese Weise ein Stäubemittel, das 3 Gew.-% des Wirkstoffs enthält.

III 30 Gewichtsteile der Verbindung des Beispiels 41 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV 40 Gewichtsteile der Verbindung des Beispiels 42 werden mit 10 Teilen Natriumsalz eines Phenolsulfonsäure-harnstoff-formaldehyd-kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.-% Wirkstoff enthält.

V 20 Teile der Verbindung des Beispiels 50 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-kondensats und 68 Teilen eines paraffinischen Mineralöls innig vermischt. Man erhält eine stabile ölige Dispersion.

VI Man vermischt 90 Gewichtsteile der Verbindung des Beispiels 40 mit 10 Gewichtsteilen N-Methyl-$\alpha$-pyrrolidon und erhält eine Lösung, die zur Anwendung in Form kleinster Tropfen geeignet ist.

VII 20 Gewichtsteile der Verbindung des Beispiels 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-momoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.

VIII 20 Gewichtsteile der Verbindung des Beispiels 39 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsprodukts von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

IX 20 Gewichtsteile der Verbindung des Beispiels 39 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsprodukts von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

## Beispiele zur herbiciden Wirkung

Die Wirkung von Vertretern der neuen Verbindungen auf das Wachstum von erwünschten und unerwünschten Pflanzen wird anhand von Gewächshausversuchen vorgeführt:

Als Kulturgefäße dienten Plastikblumentöpfe mit 300 $cm^3$ Inhalt und lehmigem Sand mit etwa 1,5 % Humus als Substrat. Bei Soja wurde zusätzlich etwas Torfmull beigemischt, um ein einwandfreies Wachstum zu gewährleisten. Die Samen der Testpflanzen wurden nach Arten getrennt flach eingesät. Unmittelbar danach erfolgte die Vorauflaufapplikation der Wirkstoffe, und zwar je 1,0 kg/ha auf die Erdoberfläche. Als bekanntes Vergleichsmittel diente das bereits erwähnte N-Allyl-chloressigsäurecyclohexylamid. Sie wurden hierbei in Wasser als Verteilungsmittel suspendiert oder emulgiert und mittels fein verteilender Düsen gespritzt. Nach dem Aufbringen der Mittel wurden die Gefäße leicht beregnet, um Keimung und Wachstum in Gang zu bringen. Danach deckte man die Gefäße mit durchsichtigen Plastikhauben ab, bis die Pflanzen angewachsen waren. Diese Abdeckung bewirkte ein gleichmäßiges Keimen der Testpflanzen, sofern dies nicht durch die Wirkstoffe beeinträchtigt wurde. Die Aufstellung der Versuche erfolgte im Gewächshaus, wobei für wärmeliebende Arten wärmere Bereiche (20 bis 30°C) und für solche gemäßigter Klimate 15 bis 25°C bevorzugt wurden. Die Versuchsperiode erstreckte sich über 3 bis 4 Wochen.

Während dieser Zeit wurden die Pflanzen gepflegt und ihre Reaktion auf die einzelnen Behandlungen ausgewertet. Bewertet wurde nach einer Skala von 0 bis 100. Dabei bedeutet 0 keine Schädigung oder normaler Ablauf und 100 kein Aufgang der Pflanzen bzw. völlige Zerstörung zumindest der oberirdischen Teile.

Bei der Prüfung auf selektive herbizide Wirkung bei Vorauflaufanwendung im Gewächshaus mit der Aufwandmenge von 1,0 kg Wirkstoff je ha zeigten die Verbindungen der Beispiele 3, 42 und 50 und ebenso die Verbindungen der Beispiele 40, 41, 44 und 48 eine wesentlich bessere herbizide Aktivität als die bekannte Vergleichsverbindung. Gleichzeitig liegen in diesen Beispielen gute Verträglichkeiten vor für Kulturpflanzen wie Raps, Soja, Baumwolle einerseits sowie Raps und Weizen bei der zweiten Gruppe der oben genannten Verbindungen.

In weiteren Versuchen zur selektiven herbiziden Wirkung bei Vorauflaufanwendung im Gewächshaus zeigten die Verbindungen der Beispiele 55, 56, 16, 23, 51 und 63 eine bessere herbizide Aktivität gegen unerwünschte Gräser (Aufwandmengen von 0,5 bis 2,0 kg/ha je nach Wirkstoff) ohne breitblättrige Beispielskulturpflanzen wie Zuckerrüben, Raps und Baumwolle zu schädigen.

Ebenso wirkten in diesen Kulturen die Verbindungen der Beispiele 157, 158 selektiv gegen unerwünschte Gräser.

Weitere biologische Beispiele zeigten die Bekämpfung von unerwünschten Gräsern bei Vorauflaufanwendung im Gewächshaus der Verbindungen Nr. 23 und 157 in Sojabohnenkulturen mit je 0,5 und 2,0 kg Wirkstoff/ha.

Weitere Gewächshausversuche ergaben ferner, daß sich beispielsweise Setaria spp. in Weizenkulturen bei Vorauflaufanwendung von 0,5 kg Wirkstoff/ha der Verbindungen Nr. 55, 56 und 23 bekämpfen läßt.

Insbesondere wirken die neuen Substanzen gegen folgende Unkräuter:

| Botanischer Name | Deutscher Name |
|---|---|
| Amaranthus retroflexus | zurückgekrümmter Fuchsschwanz |
| Digitaria sanguinalis | Blut-Fingerhirse |
| Echinochloa crus-galli | Hühnerhirse |
| Lolium multiflorum | Ital. Raygras |

Fortsetzung

| Botanischer Name | Deutscher Name |
|---|---|
| Matricaria spp. | Kamille |
| Setaria spp. | Borstenhirse |

In den hier aufgeführten Beispielen wurden die Kulturpflanzen und die unerwünschten Pflanzen vor dem Auflaufen behandelt. Die Anwendung kann aber auch erfolgen, wenn die Kulturpflanzen zwar schon etabliert sind, die Unkräuter und Ungräser aber noch nicht aufgelaufen sind oder diese sich gerade beim Auflaufen oder in frühen Jugendstadien (Nachlaufanwendung) befinden. Sind hierbei gewisse bereits stehende Kulturpflanzen gegenüber den Wirkstoffen etwas empfindlich, so können auch Ausbringungstechniken angewandt werden, bei welchen die herbiziden Mittel mit Hilfe der Spritzgeräte so geleitet werden, daß die Blätter empfindlicher Kulturpflanzen nach Möglichkeit nicht getroffen werden, während sie auf die unbedeckte Bodenfläche gelangen und die darin keimenden und aufwachsenden unerwünschten Pflanzen erreichen (post-directed, lay-by, Unterblattspritzung).

In Anbetracht der guten Verträglichkeit und der Vielseitigkeit der Applikationsmethoden und Formulierungsmöglichkeit können die erfindungsgemäßen Herbizide noch in großer Zahl von Kulturpflanzen zur Beseitigung von unerwünschten Pflanzen eingesetzt werden. Die Aufwandmengen können dabei je nach Bodenart und Bekämpfungsvorhaben zwischen 0,1 und 10 kg/ha und mehr schwanken.

In Betracht kommen beispielsweise folgende Kulturen:

| Botanischer Name | Deutscher Name | Englicher Name |
|---|---|---|
| Allium cepa | Küchenzwiebel | onions |
| Ananas comosus | Ananas | pineapple |
| Arachis hypogaea | Erdnuß | peanuts (groundnuts) |
| Asparagus officinalis | Spargel | asparagus |
| Beta vulgaris spp. altissima | Zuckerrübe | sugarbeets |
| Beta vulgaris spp. rapa | Futterrübe | fodder beets |
| Beta vulgaris spp. esculenta | Rote Rübe | table beets, red beets |
| Brassica napus var. napus | Raps | rape seed |
| Brassica napus var. napobrassica | Kohlrübe | |
| Brassica napus var. rapa | Weiße Rübe | turnips |
| Brassica rapa var. silvestris | Rübsen | |
| Camellia sinensis | Teestrauch | tea plants |
| Carthamus tinctorius | Saflor – Färberdistel | safflower |
| Carya illinoinensis | Pekannußbaum | pecan trees |
| Cirus limon | Zitrone | lemon |
| Citrus maxima | Pampelmuse | grapefruits |
| Citrus reticulata | Mandarine | |

Fortsetzung

| Botanischer Name | Deutscher Name | Englicher Name |
|---|---|---|
| Citrus sinensis | Apfelsine, Orange | orange trees |
| Coffea arabica (Coffea canephora, Coffea liberica) | Kaffee | coffee plants |
| Cucumis melo | Melone | melons |
| Cucumis sativus | Gurke | cucumber |
| Cynodon dactylon | Bermudagras | Bermudagrass in turfs and lawns |
| Daucus carota | Möhre | carrots |
| Elaeis guineensis | Ölpalme | oil palms |
| Fragaria vesca | Erdbeere | strawberries |
| Glycine max | Sojabohne | soybeans |
| Gossypium hirsutum (Gossypium arboreum, Gossypium herbaceum, Gossypium vitifolium) | Baumwolle | cotton |
| Helianthus annuus | Sonnenblume | sunflowers |
| Helianthus tuberosus | Topinambur | |
| Hevea brasiliensis | Parakautschukbaum | rubber plants |
| Hordeum vulgare | Gerste | barley |
| Humulus lupulus | Hopfen | hop |
| Ipomoea batatas | Süßkartoffeln | sweet potato |
| Juglans regia | Walnußbaum | walnut trees |
| Lactuca sativa | Kopfsalat | lettuce |
| Lens culinaris | Linse | lentils |
| Linum usitatissimum | Faserlein | flax |
| Lycopersicon lycopersicum | Tomate | tomato |
| Malus spp. | Apfel | apple trees |
| Manihot esculenta | Maniok | cassava |
| Medicago sativa | Luzerne | alfalfa (lucerne) |
| Mentha piperita | Pfefferminze | peppermint |
| Musa spp. | Obst- und Mehlbanane | banana plants |
| Nicotiana tabacum (N. rustica) | Tabak | tabacco |

Fortsetzung

| Botanischer Name | Deutscher Name | Englicher Name |
|---|---|---|
| Olea europaea | Ölbaum | olive trees |
| Oryza sativa | Reis | rice |
| Phaseolus lunatus | Mondbohne | limabeans |
| Phaseolus mungo | Erdbohne | mungbeans |
| Phaseolus vulgaris | Buschbohnen | snapbeans, green beens, dry beans |
| Petroselinum crispum spp. tuberosum | Wurzelpetersilie | parsley |
| Picea abies | Rotfichte | Norway spruce |
| Abies alba | Weißtanne | fire |
| Pinus spp. | Kiefer | pine trees |
| Pisum sativum | Gartenerbse | English peas |
| Prunus avium | Süßkirsche | cherry trees |
| Prunus domestica | Pflaume | plum trees |
| Prunus dulcis | Mandelbaum | almond trees |
| Prunus persica | Pfirsich | peach trees |
| Pyrus communis | Birne | pear trees |
| Ribes sylvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sesamum indicum | Sesam | sesame |
| Solanum tuberosum | Kartoffel | Irish potatoes |
| Sorghum bicolor (s. vulgare) | Mohrehirse | sorghum |
| Sorghum dochna | Zuckerhirse | |
| Spinacia oleracea | Spinat | spinach |
| Theobroma cacao | Kakaobaum | cacao plants |
| Trifolium pratense | Rotklee | red clover |
| Triticum aestivum | Weizen | wheat |
| Vaccinium corymbosum | Kulturheidelbeere | blueberry |

Fortsetzung

| Botanischer Name | Deutscher Name | Englicher Name |
|---|---|---|
| Vaccinium vitis-idaea | Preißelbeere | cranberry |
| Vicia faba | Pferdebohnen | tick beans |
| Vigna sinensis (V. unguiculata) | Kuhbohne | cow peas |
| Vitis vinifera | Weinrebe | grapes |
| Zea mays | Mais | Indian corn, sweet corn, maize |

Zur Verbreiterung des Wirkungsspektrums und zur Erzielung synergistischer Effekte können die neuen Verbindungen mit zahlreichen Vertretern anderer herbizider oder wachstumsregulierender Wirkstoffgruppen gemischt und gemeinsam ausgebracht werden. Beispielsweise kommen als Mischungspartner Diazine, 4 H,3,1-Benzoxaïnderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht. Eine Reihe von Wirkstoffen, welche zusammen mit den neuen Verbindungen für verschiedenste Anwendungsbereiche sinnvolle Mischungen ergeben, werden beispielhaft aufgeführt:

5-Amino-4-chlor-2-phenyl-3(2 H)-pyridazinon
5-Amino-4-brom-2-phenyl-3(2 H)-pyridazinon
5-Amino-4-chlor-2-cyclohexyl-3(2 H)-pyridazinon
5-Amino-4-brom-2-cyclohexyl-3(2 H)-pyridazinon

5-Methylamino-4-chlor-2-(3-trifluormethylphenyl)-3(2 H)-pyridazinon
5-Methylamino-4-chlor-2-(3-α,α,β,β-tetrafluorethoxyphenyl)-3(2 H)-pyridazinon
5-Dimethylamino-4-chlor-2-phenyl-3(2 H)-pyridazinon
4,5-Dimethoxy-2-phenyl-3(2 H)-pyridazinon
4,5-Dimethoxy-2-cyclohexyl-3(2 H)-pyridazinon
4,5-Dimethoxy-2-(3-trifluormethylphenyl)-3(2 H)-pyridazinon
5-Methoxy-4-chlor-2-(3-trifluormethylphenyl)-3(2 H)-pyridazinon
5-Amino-4-brom-2-(3-methylphenyl)-3(2 H)-pyridazinon

3-(1-Methylethyl)-1 H-2,1,3-benzothiadiazin-4(3 H)-on-2,2-dioxid und Salze
3-(1-Methylethyl)-8-chlor-1 H-2,1,3-benzothiadiazin-4(3 H)-on-2,2-dioxid und Salze
3-(1-Methylethyl)-8-fluor-1 H-2,1,3-benzothiadiazin-4(3 H)-on-2,2-dioxid und Salze
3-(1-Methylethyl)-8-methyl-1 H-2,1,3-benzothiadiazin-4(3 H)-on-2,2-dioxid und Salze

1-Methoxymethyl-3-(1-methylethyl)-2,1,3-benzothiadiazin-4(3 H)-on-2,2-dioxid
1-Methoxymethyl-8-chlor-3-(1-methylethyl)-2,1,3-benzothiadiazin-4(3 H)-on-2,2-dioxid
1-Methoxymethyl-8-fluor-3-(1-methylethyl)-2,1,3-benzothiadiazin-4(3 H)-on-2,2-dioxid
1-Cyan-8-chlor-3-(1-methylethyl)-2,1,3-benzothiadiazin-4(3 H)-on-2,2-dioxid
1-Cyan-8-fluor-3-(1-methylethyl)-2,1,3-benzothiadiazin-4(3 H)-on-2,2-dioxid
1-Cyan-8-methyl-3-(1-methylethyl)-2,1,3-benzothiadiazin-4(3 H)-on-2,2-dioxid
1-Cyan-3-(1-methylethyl)-2,1,3-benzothiadiazin-4(3 H)-on-2,2-dioxid
1-Azidomethyl-3-(1-methylethyl)-2,1,3-benzothiadiazin-4(3 H)-on-2,2-dioxid
3-(1-methylethyl)-1 H-(pyridino-[3,2-e]2,1,3-thiadiazin-(4)-on-2,2-dioxid

N-(1-Ethylpropyl)-2,6-dinitro-3,4-dimethylanilin
N-(1-Methylethyl)-N-ethyl-2,6-dinitro-4-trifluormethyl-anilin
N-n.Propyl-N-β-chlorethyl-2,6-dinitro-4-trifluormethyl-anilin
N-n.Propyl-N-cycloproyplmethyl-2,6-dinitro-4-trifluor-methyl-anilin
N-Bis-(n.propyl)-2,6-dinitro-3-amino-4-trifluormethylanilin
N-Bis-(n.propyl)-2,6-dinitro-4-methyl-anilin
N-Bis-(n.propyl)-2,6-dinitro-4-methylsulfonyl-anilin
N-Bis(n.propyl)-2,6-dinitro-4-aminosulfonyl-anilin
Bis-(β-chlorethyl)-2,6-dinitro-4-methyl-anilin
N-Ethyl-N-(2-methylallyl)-2,6-dinitro-4-trifluormethyl-anilin

N-Methylcarbaminsäure-3,4-dichlorbenzylester
N-Methylcarbaminsäure-2,6-di(tert.butyl)-4-methylphenyl-ester
N-Phenylcarbaminsäure-isopropylester
N-3-Fluorphenylcarbaminsäure-3-methoxypropyl-2-ester
N-3-Chlorphenylcarbaminsäure-isopropylester
N-3-Chlorphenylcarbaminsäure-butin-1-yl-3-ester
N-3-Chlorphenylcarbaminsäure-4-chlor-butin-2-yl-1-ester
N-3,4-Dichlorphenylcarbaminsäure-methylester
N-(4-Amino-benzolsulfonyl)-carbaminsäure-methylester
O-(N-Phenylcarbamoyl)-propanonoxim
N-Ethyl-2-(phenylcarbamoyl)-oxypropionsäureamid
3'-N-Isopropyl-carbamoyloxy-propionsäureanilid

Ethyl-N-(3-(N'-phenylcarbamoyloxy)-phenyl)-carbamat
Methyl-N-(3-(N'-methyl-N'-phenylcarbamoyloxy)-phenyl)-carbamat
Isopropyl-N-(3-(N'-ethyl-N'-phenylcarbamoyloxy)-phenyl)-carbamat
Methyl-N-(3-(N'-3-methylphenylcarbamoyloxy)-phenyl)-carbamat
Methyl-N-(3-(N'-fluorphenylcarbamoyloxy)-phenyl)-carbamat
Methyl-N-(3-(N'-3-chlor-4-fluorphenylcarbamoyloxy)-phenyl)-carbamat
Ethyl-N-(3-N'-3-chlor-4-fluorphenylcarbamoyloxy)-phenyl)-carbamat
Ethyl-N-(3-N'-3,4-difluorphenylcarbamoyloxy)-phenyl)-carbamat
Methyl-N-(3-(N'-3,4-difluorphenylcarbamoyloxy)-phenyl)-carbamat

N-3-(4-Fluorphenoxycarbonylamino)-phenylcarbaminsäure-methylester
N-3-(2-Methylphenoxycarbonylamino)-phenylcarbaminsäure-ethylester
N-3-(4-Fluorphenoxycarbonylamino)-phenylthiolcarbaminsäure-methylester
N-3-(2,4,5-Trimethylphenoxycarbonylamino)-phenylthiolcarbaminsäure-methylester
N-3-(Phenoxycarbonylamino)-phenylthiolcarbaminsäure-methylester

N,N-Diethyl-thiolcarbaminsäure-p-chlorbenzylester
N,N-Di-n-propyl-thiolcarbaminsäure-ethylester
N,N-Di-n-propyl-thiolcarbaminsäure-n-propylester
N,N-Di-isopropyl-thiolcarbaminsäure-2,3-dichlorallylester
N,N-Di-isopropyl-thiolcarbaminsäure-2,3,3-trichlorallylester
N,N-Di-isopropyl-thiolcarbaminsäure-3-methyl-5-isoxazolyl-methylester
N,N-Di-isopropyl-thiolcarbaminsäure-3-ethyl-5-isoxazolyl-methylester
N,N-Di-sec.butyl-thiolcarbaminsäure-ethylester
N,N-Di-sec.butyl-thiolcarbaminsäure-benzylester
N-Ethyl-N-cyclohexyl-thiolcarbaminsäure-ethylester
N-Ethyl-N-bicyclo-[2,2,1]-heptyl-thiolcarbaminsäure-ethylester
S-(2,3-Dichlorallyl)-(2,2,4-trimethyl-azetidin)-1-carbothiolat
S-(2,3,3-Trichlorallyl)-2,2,4-trimethyl-azetidin)-1-carbothiolat
S-Ethyl-hexahydro-1-H-azepin-1-carbothiolat
S-Benzyl-3-methylhexahydro-1-H-azepin-1-carbothiolat
S-Benzyl-2,3-dimethylhexahydro-1-H-azepin-1-carbothiolat
S-Ethyl-3-methylhexahydro-1-H-azepin-1-carbothiolat

N-Ethyl-N-n-butyl-thiolcarbaminsäure-n.propylester
N,N-Dimethyl-dithiocarbaminsäure-2-chlorallylester
N-Methyl-dithiocarbaminsäure-Natrium
Trichloressigsäure-Natriumsalz
α,α-Dichlorpropionsäure-Natriumsalz
α,α-Dichlorbuttersäure-Natriumsalz
α,α,β,β-Tetrafluorpropionsäure-Natriumsalz
α-Methyl,α,β-dichlorpropionsäure-Natriumsalz
α-Chlor-β-(4-chlorphenyl)-propionsäure-methylester
α,β-Dichlor-β-phenylpropionsäure-methylester
Benzamido-oxy-essigsäure
2,3,5-Triiodbenzoesäure (Salze, Ester, Amide)
2,3,6-Trichlorbenzoesäure (Salze, Ester, Amide)
2,3,5,6-Tetrachlorbenzoesäure (Salze, Ester, Amide)
2-Methoxy-3,6-dichlorbenzoesäure (Salze, Ester, Amide)
2-Methoxy-3,5,6-trichlorbenzoesäure (Salze, Ester, Amide)
3-Amino-2,5,6-trichlorbenzoesäure (Salze, Ester, Amide)
O,S-Dimethyl-tetrachlor-thioterephthalat

Dimethyl-2,3,5,6-tetrachlor-terephthalat
Dinatrium-3,6-endoxohexahydro-phthalat
4-Amino-3,5,6-trichlor-picolinsäure (Salze)
2-Cyan-3-(N-methyl-N-phenyl)-amino-acrylsäureethylester
2-[4-(4'-Chlorphenoxy)-phenoxy]-propionsäureisobutylester
2-[4-(2',4'-Dichlorphenoxy)-phenoxy]-propionsäuremethylester
2-[4-(4'-Trifluormethylphenoxy)-phenoxy]-propionsäure-methylester
2-[4-(2'-Chlor-4'-trifluorphenoxy)-phenoxy]-propionsäure-Natriumsalz
2-[4-(3',5'-Dichlorpyridyl-2-oxy)-phenoxy]-propionsäure-Natriumsalz

2-(N-Benzoyl-3,4-dichlorphenylamino)-propionsäureethylester
2-(N-Benzoyl-3-chlor-4-fluorphenylamino)-propionsäure-methylester
2-(N-Benzoyl-3-chlor-4-fluorphenylamino)-propionsäureisopropylester

2-Chlor-4-ethylamino-6-isopropylamino-1,3,5-triazin
2-Chlor-4-ethylamino-6-(amino-2'-propionitril)-1,3,5-triazin
2-Chlor-4-ethylamino-6-2-methoxypropyl-2-amino-1,3,5-triazin
2-Chlor-4-ethylamino-6-butin-1-yl-2-amino-1,3,5-triazin
2-Chlor-4,6-bisethylamino-1,3,5-triazin
2-Chlor-4,6-bisisopropylamino-1,3,5-triazin
2-Chlor-4-isopropylamino-6-cyclopropylamino-1,3,5-triazin

2-Azido-4-methylamino-6-isopropylamino-1,3,5-triazin
2-Methylthio-4-ethylamino-6-isopropylamino-1,3,5-triazin
2-Methylthio-4-ethylamino-6-tert.butylamino-1,3,5-triazin
2-Methylthio-4,6-bisethylamino-1,3,5-triazin
2-Methylthio-4,6-bisisopropylamino-1,3,5-triazin

2-Methoxy-4-ethylamino-6-isopropylamino-1,3,5-triazin
2-Methoxy-4,6-bisethylamino-1,3,5-triazin
2-Methoxy-4,6-bisisopropylamino-1,3,5-triazin
4-Amino-6-tert.butyl-3-methylthio-4,5-dihydro-1,2,4-triazin-5-on
4-Amino-6-phenyl-3-methyl-4,5-dihydro-1,2,4-triazin-5-on
4-Isobutylidenamino-6-tert.butyl-3-methylthio-4,5-dihydro-1,2,4-triazin-5-on
1-Methyl-3-cyclohexyl-6-dimethylamino-1,3,5-triazin-2,4-dion

3-tert.Butyl-5-chlor-6-methyluracil
3-tert.Butyl-5-brom-6-methyluracil
3-Isopropyl-5-brom-6-methyluracil
3-sec.Butyl-5-brom-6-methyluracil
3-(2-Tetrahydropyranyl)-5-chlor-6-methyluracil
3-(2-Tetrahydropyranyl)-5,6-trimethylenuracil
3-Cyclohexyl-5,6-trimethylenuracil

2-Methyl-4-(3'-trifluormethylphenyl)-tetrahydro-1,2,4-oxadiazin-3,5-dion
2-Methyl-4-(4'-fluorphenyl)-tetrahydro-1,2,4-oxadiazin-3,5-dion
3-Amino-1,2,4-triazol
1-Allyloxy-1-(4-bromphenyl)-2-[1',2',4'-triazolyl-(1')]-ethan (Salze)
1-(4-Chlorphenoxy-3,3-dimethyl-1-(1 H-1,2,4-triazol-1-yl)-2-butanon
N,N-Diallylchloracetamid
N-Isopropyl-2-chloracetanilid
N-(1-Methyl-propin-2-yl)-2-chloracetamid

2-Methyl-6-ethyl-N-(propargyl)-2-chloracetanilid
2-Methyl-6-ethyl-N-(ethoxymethyl)-2-chloracetanilid
2-Methyl-6-ethyl-N-(2-methoxy-1-methylethyl)-2-chloracetanilid
2-Methyl-6-ethyl-N-(isopropoxycarbonylethyl)-2-chloracetanilid
2-Methyl-6-ethyl-N-(4-methoxypyrazol-1-yl-methyl)-2-chlor-acetanilid
2-Methyl-6-ethyl-N-(pyrazol-1-yl-methyl)-2-chloracetanilid
2,6-Dimethyl-N-(pyrazol-1-yl-methyl)-2-chloracetanilid
2,6-Dimethyl-N-(4-methylpyrazol-1-yl-methyl)-2-chloracetanilid
2,6-Dimethyl-N-(1,2,4-triazol-1-yl-methyl)-2-chloracetanilid
2,6-Dimethyl-N-(3,5-dimethylpyrazol-1-yl-methyl)-2-chloracetanilid
2,6-Dimethyl-N-(1,3-dioxolan-2-yl-methyl)-2-chloracetanilid

2,6-Dimethyl-N-(2-methoxyethyl)-2-chloracetanilid
2,6-Dimethyl-N-(isobutoxymethyl)-2-chloracetanilid
2,6-Diethyl-N-(methoxymethyl)-2-chloracetanilid
2,6-Diethyl-N-(n-butoxymethyl)-2-chloracetanilid
2,6-Diethyl-N-(ethoxycarbonylmethyl)-2-chloracetanilid
2,3,6-Trimethyl-N-(pyrazol-1-yl-methyl)-2-chloracetanilid
2,3-Dimethyl-N-(isopropyl)-2-chloracetanilid
2,6-Diethyl-N-(2-n-propoxyethyl)-2-chloracetanilid

2-(2-Methyl-4-chlorphenoxy-N-methoxy-acetamid
2-(α-Naphthoxy)-N,N-diethylpropionamid
2,2-Diphenyl-N,N-dimethylacetamid
α-(3,4,5-Tribrompyrazol-1-yl)-N,N-dimethylpropionamid
N-(1,1-Dimethylpropinyl)-3,5-dichlorbenzamid
N-1-Naphthylphthalamidsäure
Propionsäure-3,4-dichloranilid
Cyclopropancarbonsäure-3,4-dichloranilid
Methacrylsäure-3,4-dichloranilid
2-Methylpentancarbonsäure-3,4-dichloranilid
5-Acetamido-2,4-dimethyltrifluormethan-sulfonanilid
5-Acetamido-4-methyl-trifluormethan-sulfonanilid

2-Propionyl-amino-4-methyl-5-chlor-thiazol
O-(Methylsulfonyl)-glykolsäure-N-ethoxymethyl-2,6-dimethylanilid
O-(Methylaminosulfonyl)-glykolsäure-N-isopropyl-anilid
O-(i-Propylaminosulfonyl)-glykolsäure-N-butin-1-yl-3-anilid
O-(Methylaminosulfonyl)-glykolsäure-hexamethylenimid
2,6-Dichlor-thiobenzamid
2,6-Dichlorbenzonitril
3,5-Dibrom-4-hydroxy-benzonitril (Salze)
3,5-Diiod-4-hydroxy-benzonitril (Salze)
3,5-Dibrom-4-hydroxy-O-2,4-dinitrophenylbenzaldoxim (Salze)
3,5-Dibrom-4-hydroxy-O-2-Cyan-4-nitrophenylbenzaldoxim (Salze)
Pentachlorphenyl-Natriumsalz
2,4-Dichlorphenyl-4'-nitrophenylether
2,4,6-Trichlorphenyl-4'-nitrophenylether
2-Fluor-4,6-dichlorphenyl-4'-nitrophenylether
2-Chlor-4-trifluormethylphenyl-4'-nitrophenylether
2,4'-Dinitro-4-trifluormethyl-diphenylether
2,4-Dichlorphenyl-3'-methoxy-4'-nitro-phenylether
2-Chlor-4-trifluormethylphenyl-3'-ethoxy-4'-nitro-phenylether
2-Chlor-4-trifluormethylphenyl-3'-carboxy-4'-nitro-phenylether (Salze)
2,4-Dichlorphenyl-3'-methoxycarbonyl-4'-nitro-phenylether
2-(3,4-Dichlorphenyl)-4-methyl-1,2,4-oxadiazolidin-3,5-dion
2-(3-tert.Butylcarbamoyl-oxyphenyl)-4-methyl-1,2,4-oxadiazolidin-3,5-dion
2-(3-iso-Propylcarbamoyl-oxyphenyl)-4-methyl-1,2,4-oxadiazolidin-3,5-dion
2-Phenyl-3,1-benzoxazinon-(4)
(4-Bromphenyl)-3,4,5,9,10-pentaazatetracyclo-[5,4,1,$0^{2,6}$,$0^{8,11}$]-dodeca-3,9-dien
2-ethoxy-2,3-dihydro-3,3-dimethyl-5-benzofuranyl-methan-sulfonat
2-ethoxy-2,3-dihydro-3,3-dimethyl-5-benzofuranyl-dimethyl-aminosulfonat
2-Ethoxy-2,3-dihydro-3,3-dimethyl-5-benzofuranyl-(N-methyl-N-acetyl)-aminosulfonat
3,4-Dichlor-1,2-benzisothiazol
N-4-Chlorphenyl-allylbernsteinsäureimid
2-Methyl-4,6-dinitrophenol (Salze, Ester)
2-sec.Butyl-4,6-dinitrophenol (Salze, Ester)
2-sec.Butyl-4,6-dinitrophenol-acetat
2-tert.Butyl-4,6-dinitrophenol-acetat

2-tert.Butyl-4,6-dinitrophenol (Salze)
2-tert.Butyl-5-methyl-4,6-dinitrophenol (Salze)
2-tert.Butyl-5-methyl-4,6-dinitrophenol-acetat

2-sec.Amyl-4,6-dinitrophenol (Salze, Ester)
1-(α,α-Dimethylbenzyl)-3-(4-methylphenyl)-harnstoff
1-Phenyl-3-(2-methylcyclohexyl)-harnstoff

1-Phenyl-1-benzoyl-3,3-dimethyl-harnstoff
1-(4-chlorphenyl)-1-benzoyl-3,3-dimethyl-harnstoff
1-(4-chlorphenyl)-3,3-dimethyl-harnstoff
1-(4-Chlorphenyl)-3-methyl-3-butin-1-yl-3-harnstoff
1-(3,4-Dichlorphenyl)-3,3-dimethyl-harnstoff
1-(3,4-Dichlorphenyl)-1-benzoyl-3,3-dimethyl-harnstoff
1-(3,4-Dichlorphenyl)-3-methyl-3-n-butyl-harnstoff
1-(4-i-Propylphenyl)-3,3-dimethyl-harnstoff
1-(3-Trifluormethylphenyl)-3,3-dimethyl-harnstoff
1-(α,α,β,β-Tetrafluoräthoxyphenyl)-3,3-dimethyl-harnstoff

1-(3-tert.Butylcarbamoyloxy-phenyl)-3,3-dimethyl-harnstoff
1-(3-Chlor-4-methylphenyl)-3,3-dimethyl-harnstoff
1-(3-Chlor-4-methoxyphenyl)-3,3-dimethyl-harnstoff
1-(3,5-Dichlor-4-methoxyphenyl)-3,3-dimethyl-harnstoff
1-[4(4'-Chlorphenoxy)-phenyl]-3,3-dimethyl-harnstoff
1-[4(4'-methoxyphenoxy)-phenyl]-3,3-dimethyl-harnstoff
1-Cyclooctyl-3,3-dimethyl-harnstoff
1-(Hexahydro-4,7-methanindan-5-yl)-3,3-dimethyl-harnstoff
1-(1- oder 2-(3a,4,5,7,7a-Hexahydro)-4,7-methanoindanyl]-3,3-dimethyl-harnstoff
1-(4-Fluorphenyl)-3-carboxymethoxy-3-methyl-harnstoff
1-Phenyl-3-methyl-3-methoxy-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff
1-(3-Chlor-4-isopropylphenyl)-3-methyl-3-methoxy-harnstoff
1-(3-Chlor-4-methoxyphenyl)-3-methyl-3-methoxy-harnstoff
1-(3-tert.Butylphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethyl-harnstoff
Imidazolidin-2-on-1-carbonsäure-iso-butylamid
1,2-Dimethyl-3,5-diphenylpyrazolium-methylsulfat
1,2-4-Trimethyl-3,5-diphenylpyrazolium-methylsulfat
1,2-Dimethyl-4-brom-3,5-diphenylpyrazolium-methylsulfat
1,3-Dimethyl-4-(3,4-dichlorbenzoyl)-5-[(4-methylphenylsulfonyl)-oxy]-pyrazol
2,3,5-Trichlor-pyridinol-(4)
1-Methyl-3-phenyl-5-(3'-trifluormethylphenyl)-pyridon-(4)
1-Methyl-4-phenyl-pyridiniumchlorid
1,1-Dimethylpyridiniumchlorid
3-Phenyl-4-hydroxy-6-chlorpyridazin
1,1'-Dimethyl-4,4'-dipyridylium-di-(methylsulfat)
1,1'-Di(3,5-dimethylmorpholin-carbonylmethyl)-4,4'-di-pyridylium-dichlorid
1,1'-Ethylen-2,2'-dipyridylium-dibromid
3-[1(N-Ethoxyamino)-propyliden]-6-ethyl-3,4-dihydro-2-H-pyran-2,4-dion
3-[1-(N-Allyloxyamino)-propyliden]-6-ethyl-3,4-dihydro-2-H-pyran-2,4-dion
2-[1-(N-Allyloxyamino)-propyliden]-5,5-dimethylcyclohexan-1,3-dion (Salze)
2-[1-(N-Allyloxyamino-butyliden]-5,5-dimethylcyclohexan-1,3-dion (Salze)
2-[1-(N-Allyloxyamino-butyliden]-5,5-dimethyl-4-methoxycarbonyl-cyclohexan-1,3-dion (Salze)
2-Chlorphenoxyessigsäure (Salze, Ester, Amide)
4-Chlorphenoxyessigsäure (Salze, Ester, Amide)
2,4-Dichlorphenoxyessigsäure (Salze, Ester, Amide)
2,4,5-Trichlorphenoxyessigsäure (Salze, Ester, Amide)
2-Methyl-4-chlorphenoxyessigsäure (Salze, Ester, Amide)
3,5,6-Trichlor-2-pyridinyl-oxyessigsäure (Salze, Ester, Amide)

α-Naphthoxyessigsäuremethylester
2-(2-Methylphenoxy)-propionsäure (Salze, Ester, Amide)
2-(4-Chlorphenoxy)-propionsäure (Salze, Ester, Amide)
2-(2,4-Dichlorphenoxy)-propionsäure (Salze, Ester, Amide)
2-(2,4,5-Trichlorphenoxy)-propionsäure (Salze, Ester, Amide)
2-(2-Methyl-4-chlorphenoxy)-propionsäure (Salze, Ester, Amide)
4-(2,4-Dichlorphenoxy)-buttersäure (Salze, Ester, Amide)
4-(2-Methyl-4-chlorphenoxy)-buttersäure (Salze, Ester, Amide)

Cyclohexyl-3-(2,4-dichlorphenoxy-acrylat
9-Hydroxyfluoren-carbonsäure-(9) (Salze, Ester)
2,3,6-Trichlorphenyl-essigsäure (Salze, Ester)
4-Chlor-2-oxo-benzothiazolin-3-yl-essigsäure (Salze, Ester)
Gibellerinsäure (Salze)
Dinatrium-methylarsonat
Mononatriumsalz der Methylarsonsäure
N-Phosphon-methyl-glycin (Salze)
N,N-Bis-(phosphonmethyl)-glycin (Salze)
2-Chlorethanphosphonsäure-2-chlorethylester
Ammonium-ethyl-carbamoyl-phosphonat
Di-n-butyl-1-n.butylamino-cyclohexyl-phosphonat
Trithiobutylphosphit
O,O-Diisopropyl-5-(2-benzosulfonylamino-ethyl)-phosphordithionat
2,3-Dihydro-5,6-dimethyl-1,4-dithiin-1,1,4,4-tetraoxid
5-tert.Butyl-3-(2,4-dichlor-5-isopropoxyphenyl)-1,3,4-oxadiazolon-(2)
4,5-Dichlor-2-trifluormethyl-benzimidazol (Salze)
1,2,3,6-Tetrahydropyridazin-3,6-dion (Salze)
Bernsteinsäure-mono-N-dimethylhydrazid (Salze)
(2-Chlorethyl)-trimethyl-ammoniumchlorid
(2-Methyl-4-phenylsulfonyl)-trifluormethansulfoanilid
1,1-Dimethyl-4,6-diisopropyl-5-indanylethylketon
Natriumchlorat
Ammoniumrhodanid
Calciumcyanamid

2-Chlor-4-trifluormethylphenyl-3'-ethoxycarbonyl-4'-nitrophenylether
1-(4-Benzyloxyphenyl)-3-methyl-3-methoxyharnstoff
2-[1-(2,5-Dimethylphenyl)-ethylsulfonyl]-pyridin-N-oxid
1-Acetyl-3-anilino-4-methoxycarbonyl-5-methylpyrazol
3-Anilino-4-methoxycarbonyl-5-methylpyrazol
3-tert.Butylamino-4-methoxycarbonyl-5-methylpyrazol
N-Benzyl-N-isopropyl-trimethylacetamid
2-[4-(4'-Chlorphenoxymethyl)-phenoxy]-propionsäuremethylester
2-[4-(5'-Brompyridyl-2-oxy)-phenoxy]-propionsäureethylester
2-[4-(5'-Iodpyridyl-2-oxy)-phenoxy]-propionsäure-n-butylester

2-Chlor-4-trifluormethylphenyl-3'-(2-fluorethoxy)-4'-nitro-phenylether
2-Chlor-4-trifluormethylphenyl-3-(ethoxycarbonyl)-methylthio-4-nitrophenylether
2,4,6-Trichlorphenyl-3-(ethoxycarbonyl)-methylthio-4-nitrophenylether
2-[1-(N-ethoxamino)-butyliden]-5-(2-ethylthiopropyl)-3-hydroxy-cyclohexen-(2)-on-(1) (Salze)
2-[1-(N-ethoxamino)-butyliden]-5-(2-phenylthiopropyl)-3-hydroxy-cyclohexen-(2)-on-(1) (Salze)

4-[4-(4'-Trifluormethyl)-phenoxy]-penten-2-carbonsäureethylester

2-Chlor-4-trifluormethyl-3'-methoxycarbonyl-4'-nitrophenylether
2,4-Dichlorphenyl-3'-carboxy-4'-nitrophenylether (Salze)
4,5-Dimethoxy-2-(3-$\alpha,\alpha,\beta$-trifluor-$\beta$-bromoethoxyphenyl)-3-(2 H)-pyridazinon
2,4-Dichlorphenyl-3'-ethoxy-ethoxy-ethoxy-4'-nitrophenyl-ether
2,3-Dihydro-3,3-dimethyl-5-benzofuranyl-ethansulfonat
N-[4-Methoxy-6-methyl-1,3,5-triazin-2-yl-aminocarbonyl]-2-chlorbenzolsulfonamid
1-(3-Chlor-4-ethoxyphenyl)-3,3-dimethylharnstoff
2-Methyl-4-Chlorphenoxy-thioessigsäureethylester
2-Chlor-3,5-diiod-4-acetoxy-pyridin
1-(4-[2-(4-Methylphenyl)-ethoxy]-phenyl)-3-methyl-3-methoxyharnstoff
2,6-Dimethyl-N-(pyrazol-1-yl-methylenoxymethyl)-2-chloracetanilid
2-Methyl-6-ethyl-N-(pyrazol-1-yl-methylenoxymethyl)-2-chloracetanilid
1-($\alpha$-2,4-Dichlorphenoxypropionsäure)-3-(O-methylcarbamoyl)-anilid
1-($\alpha$-2-Brom-4-chlorphenoxypropionsäure)-3-(O-methylcarbamoyl)-anilid
2-Methyl-6-ethyl-N-(pyrazol-1-yl-ethylenoxymethyl)-2-chloracetanilid
Methyl-N-dichlorfluormethylsulfenyl-(3-(N-dichlorfluormethylsulfenyl-N'-phenylcarbamoyloxy)-phenyl)-carbamat
Methyl-N-dichlorfluormethylsulfenyl-(3-(N'-dichlorfluormethylsulfenyl-N'-3-methylphenyl-carbamoyl-oxy)-phenyl)-carbamat
N-(Pyrazol-1-yl-methyl)-pyrazol-1-yl-essigsäure-2,6-dimethylanilid

N-(Pyrazol-1-yl-methyl)-1,2,4-triazol-1-yl-essigsäure-2,6-dimethylanilid
2-(3'-Trifluormethylphenyl)-4 H-3,1-benzoxazin-4-on
2-(2-Thienyl)-4 H-3,1-benzoxazin-4-on
2-(3-Pentafluorethoxyphenyl)-4 H-3,1-benzoxazin-4-on
2-(3-Trifluormethylthio-phenyl)-4 H-3,1-benzoxazin-4-on
2-(3-Difluor-chlormethoxyphenyl)-4 H-3,1-benzoxazin-4-on

5-Nitro-2-(3-trifluormethyl-phenyl)-4 H-3,1-benzoxazin-4-on
5-Chlor-2-(3-trifluormethoxyphenyl)-4 H-3,1-benzoxazin-4-on
5-Chlor-2-(3-$\alpha,\alpha,\beta,\beta$)-tetrafluorethoxyphenyl)-4 H-3,1-benzoxazin-4-on
5-Fluor-2-(3-$\alpha,\alpha,\beta,\beta$-tetrafluorethoxyphenyl)-4 H-3,1-benzoxazin-4-on
5-Chlor-2-(4-Difluorchlormethoxyphenyl)-4 H-3,1-benzoxazin-4-on
5-Fluor-2-(4-Difluorchlormethoxyphenyl)-4 H-3,1-benzoxazin-4-on
5-Fluor-2-(phenyl)-4 H-3,1-benzoxazin-4-on
5-Fluor-2-(3-Difluormethoxyphenyl)-4 H-3,1-benzoxazin-4-on
5-Chlor-2-(phenyl)-4 H-3,1-benzoxazin-4-on
3-(3,5-Dichlorphenyl)-4-methoxycarbonyl-5-methylpyrazol
3-(3-Chlorphenyl)-4-methoxycarbonyl-5-methylpyrazol
3-(3-Fluorphenyl)-4-methoxycarbonyl-5-methylpyrazol
1-Acetyl-3-(3-fluorphenyl)-4-methoxycarbonyl-5-methylpyrazol
1-Acetyl-3-(3-chlorphenyl)-4-methoxycarbonyl-5-methylpyrazol
1-Acetyl-3-(3-bromphenyl)-4-methoxycarbonyl-5-methylpyrazol
1-Acetyl-3-(3,5-dichlorphenyl-4-methoxycarbonyl-5-methylpyrazol
1-Acetyl-3-thienyl-4-methoxycarbonyl-5-methylpyrazol
N-3-Chlor-4-isopropylphenyl-thiolcarbaminsäuremethylester
N-3-Methyl-4-fluorphenyl-thiolcarbaminsäuremethylester
N-3-Chlor-4-isopentylphenyl-thiolcarbaminsäuremethylester
N-3-Chlor-4-difluormethoxyphenyl-thiolcarbaminsäuremethylester
N-3-Chlor-4-(1-chlorisopropyl)-phenyl-thiolcarbaminsäuremethylester
1-(2-Fluorphenyl)-3-methyl-5-iminoimidazolidin-2-on
1-(3-Isopropylphenyl)-3-methyl-5-iminoimidazolidin-2-on
1-(4-Isopropylphenyl)-3-methyl-5-iminoimidazolidin-2-on
1-[3-(1,1,2,2-Tetrafluorethoxy)-phenyl]-3-methyl-5-iminoimidazolidin-2-on
1-(3,4-Dichlorphenyl)-3-methyl-5-iminoimidazolidin-2-on
1-(3,4-Difluorphenyl)-3-methyl-5-iminoimidazolidin-2-on
6-Methyl-3-methoxy-5,6-dihydro-1,2,4,6-thiatriazin-5-on-1,1-dioxid
6-Methyl-3-methoxy-5,6-dihydro-1,2,4,6-thiatriazin-5-on-1,1-dioxid (Na-Salz)
6-n-Propyl-3-methoxy-5,6-dihydro-1,2,4,6-thiatriazin-5-on-1,1-dioxid
6-Methyl-3-ethoxy-5,6-dihydro-1,2,4,6-thiatriazin-5-on-1,1-dioxid
6-n-Propyl-3-ethoxy-5,6-dihydro-1,2,4,6-thiatriazin-5-on-1,1-dioxid (Na-Salz)
6-Methyl-3-iso-propoxy-5,6-dihydro-1,2,4,6-thiatriazin-5-on-1,1-dioxid
6-n-propyl-3-iso-propoxy-5,6-dihydro-1,2,4,6-thiatriazin-5-on-1,1-dioxid
6-iso-Propyl-3-sek.butoxy-5,6-dihydro-1,2,4,6-thiatriazin-5-on-1,1-dioxid (Na-Salz)
N-3'-(2"-Chlor-4"-trifluormethyl-phenoxy)-6'-nitrobenzoylanthranilsäure
N-3'-(2"-Chlor-4"-trifluormethyl-phenoxy)-6'-nitrobenzoylanthranilsäure-methylester
N-3'-(2"-Chlor-4"-trifluormethyl-phenoxy)-6'-nitrobenzoylanthranilsäure (Na-Salz)
N-3'-(2"-Chlor-4"-trifluormethyl-phenoxy)-6'-nitrobenzoyl-3-chloranthranilsäure
N-3'-(2"-Chlor-4"-trifluormethyl-phenoxy)-benzoyl-3-chloranthranilsäure
N-3'-(2"-Chlor-4"-trifluormethyl-phenoxy)-benzoyl-3-methylanthranilsäure
N-3'-(2"-Chlor-4"-trifluormethyl-phenoxy)-benzoylanthranilsäure
N-3'-(2",4"-Dichlorphenoxy)-6'-nitrobenzoylanthranilsäure
2-[3'-(2"-Chlor-4"-trifluormethyl-phenoxy)-6'-nitrophenyl]-4 H-1,3-benzoxazin-4-on
2-[3'-(2"-Chlor-4"-trifluormethyl-phenoxy)-6'-nitrophenyl]-4 H-1,3-8-methoxy-benzoxazin-4-on

Außerdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nichtphytotoxische Öle und Ölkonzentrate zugesetzt werden.

**Patentansprüche**

1. Chloressigsäurecyclohexylamide der Formel I

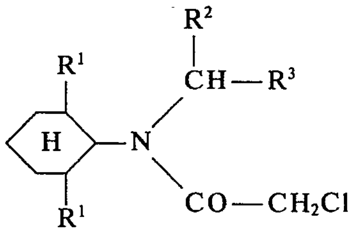

(I)

worin

$R^1$ Methyl oder Ethyl,
$R^2$ Wasserstoff oder $C_{1-3}$-Alkyl und
$R^3$ ein durch $C_{1-4}$-Alkoxi oder $C_{1-4}$-Alkoxicarbonyl substituiertes $C_{1-6}$-Alkyl, $C_{2-3}$-Alkenyl, $C_{2-3}$-Alkinyl, Furanyl, Tetrahydrofuranyl, gegebenenfalls durch Halogen substituiertes Phenyl, (Bis-$C_{1-6}$-alkoxi)-methyl, gegebenenfalls durch $C_{1-3}$-Alkyl substituiertes Dioxolanyl oder Dioxanyl oder – falls $R^2$ Wasserstoff bedeutet – auch $C_{1-8}$-Alkyloxi, $C_{3-6}$-Alkenyloxi, $C_{3-6}$-Alkinyloxi, $C_{3-6}$-Cycloalkylmethoxi, $C_{4-6}$-Cycloalkyloxi, $C_{1-6}$-Alkyloxiethoxi, Tetrahydrofuranylmethoxi, Tetrahydropyranyl methoxi oder Azol-1-yl

bedeuten.

2. Verbindungen der Formel I gemäß Anspruch 1, dadurch gekennzeichnet, daß die Substituenten $R^1$ in cis-Stellung zum N-Atom stehen.

3. Verbindungen der Formel I gemäß Anspruch 1, dadurch gekennzeichnet, daß die Substituenten $R^1$ in trans-Stellung zum N-Atom stehen.

4. Verbindungen der Formel I gemäß Anspruch 1, dadurch gekennzeichnet, daß die Substituenten $R^1$ in cis,trans-Stellung zum N-Atom stehen.

5. Verfahren zur Herstellung der Verbindungen der Formel I gemäß Anspruch 1, dadurch gekennzeichnet, daß man

a) ein N-substituiertes Cyclohexylamin der Formel II

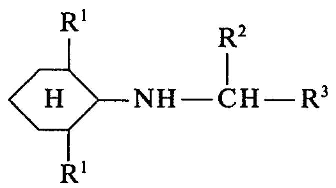

(II)

worin $R^1$, $R^2$ und $R^3$ die oben angegebene Bedeutung besitzen, mit Chloressigsäureanhydrid oder einem Chloressigsäurehalogenid umsetzt oder

b) – falls $R^2$ Wasserstoff und $R^3$ $C_{1-8}$-Alkyloxi, $C_{3-6}$-Alkenyloxi, $C_{3-6}$-Alkinyloxi, $C_{3-6}$-Cycloalkylmethoxi, $C_{4-6}$-Cycloalkyloxi, $C_{1-6}$-Alkyloxiethoxi, Tetrahydrofuranylmethoxi, Tetrahydropyranylmethoxi oder Azol-1-yl bedeuten – ein N-Chlormethylchloressigsäurecyclohexylamid der Formel III

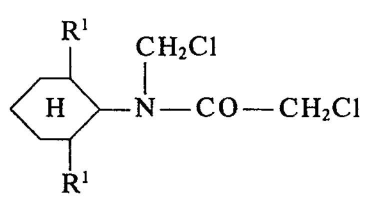

(III)

worin $R^1$ die oben angegebene Bedeutung hat,

mit einer Verbindung der Formel $R^3H$, worin $R^3$ die oben unter b) angegebene Bedeutung besitzt, umsetzt.

6. Herbizides Mittel, enthaltend mindestens eine Verbindung der Formel I gemäß Anspruch 1.

7. Herbizides Mittel enthaltend mindestens eine Verbindung der Formel I gemäß Anspruch 1 und einen festen oder flüssigen Trägerstoff.

8. Verwendung einer Verbindung der Formel I gemäß Anspruch 1 zur Bekämpfung von Unkräutern.

9. Verfahren zur Bekämpfung von Unkräutern, dadurch gekennzeichnet, daß man mindestens eine Verbindung der Formel I gemäß Anspruch 1 auf die Unkräuter oder durch diese bedrohte Flächen einwirken läßt.

**Claims**

1. Chloracetic acid cyclohexylamides of the formula I

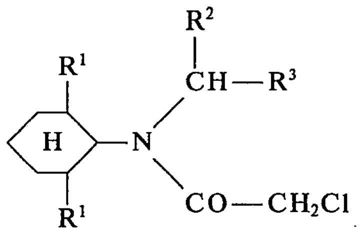

(I)

where $R^1$ is methyl or ethyl, $R^2$ is hydrogen or $C_1$—$C_3$-alkyl and $R^3$ is $C_1$—$C_6$-alkyl which is substituted by $C_1$—$C_4$-alkoxy or $C_1$—$C_4$-alkoxycarbonyl, or is $C_2$—$C_3$-alkenyl, $C_2$—$C_3$-alkynyl, furanyl or tetrahydrofuranyl, or is phenyl which is unsubstituted by halogen, or is (bis-$C_1$—$C_6$-alkoxy)-methyl, or is dioxanyl or dioxolanyl which is unsubstituted or substituted by $C_1$—$C_3$-alkyl, and if $R^2$ is hydrogen, may also be $C_1$—$C_8$-alkoxy, $C_3$—$C_6$-alkenyloxy, $C_3$—$C_6$-alkynyloxy, $C_3$—$C_6$-cycloalkylmethoxy, $C_4$—$C_6$-cycloalkoxy, $C_1$—$C_6$-alkoxyethoxy, tetrahydrofuranylmethoxy, tetrahydropyranylmethoxy or azol-1-yl.

2. Compounds of the formula I as claimed in claim 1, wherein the substituents $R^1$ are in cis-position to the nitrogen atom.

3. Compounds of the formula I as claimed in claim 1, wherein the substituents $R^1$ are in trans-position to the nitrogen atom.

4. Compounds of the formula I as claimed in claim 1, wherein the substituents $R^1$ are in cis,trans-position to the nitrogen atom.

5. A process for the manufacture of compounds of the formula I as claimed in claim 1, wherein

a) an N-substituted cyclohexylamine of the formula II

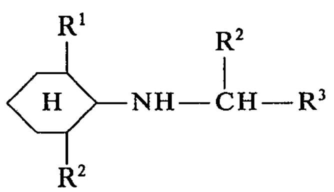

(II)

where $R^1$, $R^2$ and $R^3$ have the above meanings, is reacted with chloroacetic anhydride or a chloroacetyl halide, or

b) if $R^2$ is hydrogen and $R^3$ is $C_1$—$C_8$-alkoxy, $C_3$—$C_6$-alkenyloxy, $C_3$—$C_6$-alkynyloxy, $C_3$—$C_6$-cycloalkylmethoxy, $C_4$—$C_6$-cycloalkoxy, $C_1$—$C_6$-alkoxyethoxy, tetrahydrofuranylmethoxy, tetrahydropyranylmethoxy or azol-1-yl, a chloroacetic acid N-chloromethyl-N-cyclohexylamide of the formula III

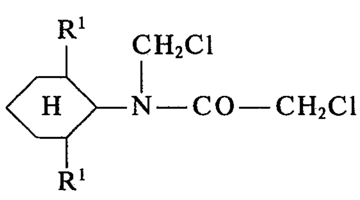

(III)

where $R^1$ has the above meanings, is reacted with a compound of the formula $R^3H$, where $R^3$ has the meanings given above under b).

6. A herbicidal agent containing at least one compound of the formula I as claimed in claim 1.

7. A herbicidal agent containing at least one compound of the formula I as claimed in claim 1 and a solid or liquid carrier.

8. The use of a compound of the formula I as claimed in claim 1 for combating weeds.

9. A process for combating weeds, wherein at least one compound of the formula I as claimed in claim 1 is allowed to act on the weeds or areas treatened by them.

## Revendications

1. Cyclohexylamide d'acide chloracétique de formule I

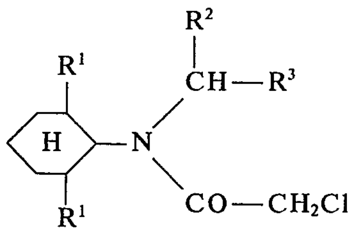

(I)

dans laquelle

$R^1$ représente méthyle ou éthyle,
$R^2$ hydrogène ou alkyle en $C_{1-3}$ et
$R^3$ alkyle en $C_{1-6}$ substitué par alcoxy en $C_{1-4}$, alcényle en $C_{2-3}$, alcinyle en $C_{2-3}$, furanyle, tétrahydrofuranyle, phényle éventuellement substitué par halogène, (bis-alcoxy en $C_{1-6}$)-méthyle, dioxolanyle ou dioxanyle éventuellement substitué par alkyle en $C_{1-3}$, ou – lorsque $R^2$ représente hydrogène – aussi alkyloxy en $C_{1-8}$, alcényloxy en $C_{3-6}$, alcinyloxy en $C_{3-6}$, cycloalkylméthoxy en $C_{3-6}$, cycloalkyloxy en $C_{4-6}$, alkyloxyméthoxy en $C_{1-6}$, tétrahydrofuranylméthoxy, tétrahydrofuranylméthoxy ou qzol-1-yle.

2. Composés de formule I selon la revendication 1, caractérisé par le fait que les substituants $R^1$ sont en position cis sur l'atome N.
3. Composés de formule I selon la revendication 1, caractérisé par le fait que les substituants $R^1$ sont en position trans sur l'atome N.
4. Composés de formule I selon la revendication 1, caractérisé par le fait que les substituants $R^1$ sont en position cis,trans- sur l'atome N.
5. Procédé de préparation de composés de formule I selon la revendication 1, caractérisé par le fait qu'on fait réagir:

a) une cyclohexylamine substituée sur N de formule II

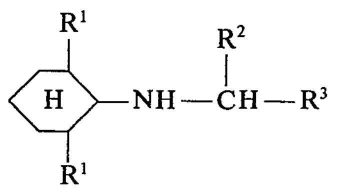

(II)

où $R^1$, $R^2$, $R^3$ ont les significations sus-indiquées, avec l'anhydride d'acide chloracétique ou un halogénure d'acide chloracétique, ou

b) lorsque $R^2$ représente hydrogène et $R^3$ alkyloxy en $C_{1-8}$, alcényloxy en $C_{3-6}$, alcinyloxy en $C_{3-6}$, cycloalkylméthoxy en $C_{4-6}$, alkyloxyéthoxy en $C_{1-6}$, tétrahydrofuranylméthoxy, tétrahydropyranylméthoxy ou azol-2-yle un cyclohexylamide d'acide N-chlorométhylchloracétique de formule III

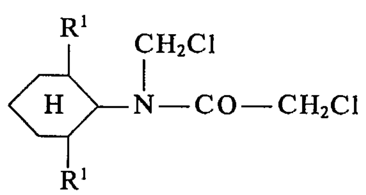

(III)

où $R^1$ a la signification sus-indiquée, avec un composé de formule $R^3H$, où $R^3$ a la signification indiquée ci-dessus sous b).

6. Herbicide contenant au moins un composé de formule I selon la revendication 1.
7. Herbicide contenant au moins un composé de formule I selon la revendication 1 et un support solide ou liquide.
8. Utilisation d'un composé de formule I selon la revendication 1 pour lutter contre les mauvaises herbes.
9. Procédé pour lutter contre les mauvaises herbes, caractérisé par le fait agir au moins un composé de formule I selon la revendication 1 sur les mauvaises herbes ou sur les surfaces menacées par celles-ci.